# EUROPEAN PATENT APPLICATION

(11) **EP 2 628 747 A1**
(43) Date of publication of application: **21.08.2013**
(21) Application number: 13153125.3
(22) Date of filing: 11.12.2008
(51) Int. Cl.: C07K 2/00, A61K 38/02, C07K 14/78

(54) **Impaired wound healing compositions and treatments**

(30) Priority: 11.12.2007 US 7323 P
(62) Divisional of application: 08860470.7
(71) Applicant: Coda Therapeutics, Inc., San Diego, CA 92121 (US)
(72) Inventor: Becker, David L., Abbots Langley, Hertfordshire WD5 0GG (GB); Green, Colin R., 1023 Epson (NZ); Duft, Bradford J., Rancho, CA California 92067 (US)
(74) Representative: Tuxworth, Pamela M.

(57) **Abstract**

Methods, compounds, compositions, kits and articles of manufacture comprising one or more gap junction modulating agents for treatment of wounds that do not heal at expected rates, including chronic wounds, delayed healing wounds, incompletely healing wounds, and dehiscent wounds in a subject in need thereof.

## Description

### FIELD

The inventions relate to gap junctions and to wounds and wound healing, in particular to wounds that do not heal at expected rates, such as delayed-healing wounds, incompletely healing wounds, chronic wounds, and dehiscent wounds.

### BRIEF BACKGROUND

The following includes information that may be useful in understanding the present invention. It is not an admission that any of the information provided herein is prior art, or relevant, to the presently described or claimed inventions, or that any publication or document that is specifically or implicitly referenced is prior art.

In humans and other mammals wound injury triggers an organized complex cascade of cellular and biochemical events that will in most cases result in a healed wound. An ideally healed wound is one that restores normal anatomical structure, function, and appearance at the cellular, tissue, organ, and organism levels. Wound healing, whether initiated by trauma, microbes or foreign materials, proceeds via a complex process encompassing a number of overlapping phases, including inflammation, epithelialization, angiogenesis and matrix deposition. Normally, these processes lead to a mature wound and a certain degree of scar formation. Although inflammation and repair mostly occur along a prescribed course, the sensitivity of the process is dependent on the balance of a variety of wound healing modulating factors, including for example, a complex network of regulatory cytokines and growth factors.

Gap junctions are cell membrane structures that facilitate direct cell-cell communication. A gap junction channel is formed of two connexons (hemichannels), each composed of six connexin subunits. Each hexameric connexon docks with a connexon in the opposing membrane to form a single gap junction. Gap junction channels are reported to be found throughout the body. Tissue such as the corneal epithelium, for example, has six to eight cell layers, yet expresses different gap junction channels in different layers with connexin 43 in the basal layer and connexin 26 from the basal to middle wing cell layers. In general, connexins are a family of proteins, commonly named according to their molecular weight or classified on a phylogenetic basis into alpha, beta, and gamma subclasses. At least 20 human and 19 murine isoforms have been identified. Different tissues and cell types are reported to have characteristic patterns of connexin protein expression and tissues such as cornea have been shown to alter connexin protein expression pattern following injury or transplantation (Qui et al. (2003) Current Biology, 13:1967-1703; Brander et al. (2004), J. Invest Dermatol. 122:1310-20).

It has been reported that abnormal connexin function may be linked to certain disease states (*e.g.* heart diseases) (A. C. de Carvalho, et al. (1994) J Cardiovasc Electrophysiol 5: 686). In certain connexin proteins, alterations in the turnover and trafficking properties may be induced by the addition exogenous agents which may affect the level of gap junctional intercellular communication (Darrow et al. (1995) Circ Res 76: 381; Lin et al. (2001) J Cell Biol 154(4):815). Antisense technology has also been reported for the modulation of the expression for genes implicated in viral, fungal and metabolic diseases. See, *e.g.,* U.S. Pat. No. 5,166,195, (oligonucleotide inhibitors of HIV), U.S. Pat. No. 5,004,810 (oligomers for hybridizing to herpes simplex virus Vmw65 mRNA and inhibiting replication). See also U.S. Pat. No. 7,098,190 to Becker and Green (formulations comprising antisense nucleotides to connexins). Peptide inhibitors of gap junctions and hemichannels have also been reported. See for example, Berthoud et al. (2000) Am J. Physiol. Lung Cell Mol. Physiol. 279:L619-L622; Evans, W.H. and Boitano, S. Biochem. Soc. Trans. 29:606-612, and De Vriese A.S., et al. (2001) Kidney Int. 61:177-185. See also Green and Becker, WO2006/134494 ("Anti-connexin compounds and methods of use").

Despite advances in the understanding of the principles underlying the wound healing process, there remains a significant unmet need in suitable therapeutic options for wound care, including wounds that do not heal at expected rates, such as delayed-healing wounds, incompletely healing wounds, and chronic wounds. Such therapeutics compositions and treatments are described and claimed herein.

### BRIEF SUMMARY

The inventions described and claimed herein have many attributes and embodiments including, but not limited to, those set forth or described or referenced in this Brief Summary. It is not intended to be all-inclusive and the inventions described and claimed herein are not limited to or by the features or embodiments identified in this Brief Summary, which is included for purposes of illustration only and not restriction.

Aspects and embodiments of this disclosure relate generally to methods of treatment of a subject for wounds that do not heal at expected rates, such as slow-healing wounds, delayed-healing wounds, incompletely healing wounds, dehiscent wounds, and chronic wounds, comprising administering a composition or formulation comprising a gap junction modulating agent to the wound. Connexin 43 gap junction modulating agents are preferred.

In one embodiment the invention is direct to a method of treating a subject having a chronic wound comprising administration of a composition comprising an effective amount of a gap junction modulating agent to the wound. Connexin 43 gap junction modulating agents are preferred. In one embodiment, the chronic wound is a pressure ulcer. In one embodiment, the chronic wound is a decubitus ulcer. In another embodiment, the chronic wound is an arterial ulcer. In another embodiment, the chronic wound is a venous ulcer. In another embodiment, the chronic wound is a venous stasis ulcer. In another embodiment, the chronic wound is a vasculitic ulcer. In yet another embodiment, the chronic wound is a diabetic ulcer, such as a diabetic foot ulcer. In a further embodiment, the chronic wound is a traumatic ulcer or a burn ulcer. In another embodiment, chronic wound is an infectious ulcer, and in another embodiment the chronic wound is characterized by ulcerations associated with pyoderma gangrenosum. In a still further embodiment, the chronic wound is a mixed ulcer. In one embodiment, the wound is a persistent epithelial defect.

In one embodiment the invention is direct to a method of treating a subject having a dehiscent wound comprising administration of a composition comprising an effective amount of a gap junction modulating agent, preferably a connexin 43 gap junction modulating agent, to the wound.

In one embodiment the subject is a mammal. In another embodiment, the subject is human. In another embodiment, the subject is a human with diabetes. In one aspect the subject is a human with type 1 diabetes. In another aspect, the subject is a human with type 2 diabetes.

In another embodiment, the mammal is a sports or pet animal. In one aspect the sports animal is a horse or dog. In another aspect the pet animal is a dog or cat.

In one embodiment the invention is direct to a method of treating a subject having a delayed healing wound which comprises administration of an effective amount of a gap junction modulating agent to the wound. In another embodiment the invention is direct to a method of treating a subject having an incompletely healing wound comprising administration of a composition comprising an effective amount of a gap junction modulating agent to the wound. Connexin 43 gap junction modulating agents are preferred.

According to a further aspect, sustained administration to the wound of an amount of a gap junction modulating for a sustained period of time is provided. Further according to this aspect, wound healing of a wound that is not healing at expected rates is promoted, including delayed healing wounds, impaired healing wounds, and chronic wounds. In one embodiment the wound is a diabetic ulcer, a diabetic foot ulcer, a vasculitic ulcer, a venous ulcer, a venous stasis ulcer, an arterial ulcer, a pressure ulcer, a decubitus ulcer, an infectious ulcer, a trauma-induced ulcer, a burn ulcer, an ulceration associated with pyoderma gangrenosum, or a mixed ulcer or ulcers. Repeat applications are included within the invention. Preferred are repeat applications until wound closure is seen to be proceeding, followed by a repeat application (or applications) in the event wound healing once again becomes stalled or delayed.

According to other aspects of the present invention wound re-epithlialization and/or formation of granulation tissue is promoted.

In an additional aspect, the present invention provides a method of promoting wound healing in a subject having a wound that is not healing at an expected rate, including a delayed healing wound or an incomplete healing wound or a chronic wound, which comprises administration of an anti-connexin 43 peptide and an anti-connexin 31.1 peptide. Repeat applications are included within the invention.

An alternative aspect of the present invention is directed to a method of promoting re-epithelialization of skin wounds which comprises administering to a subject having a wound that is not healing at the expected rate, including, for example, a delayed healing or an incompletely healing wound or a chronic wound, an gap junction modulating agent, e.g., an anti-connexin peptide in an amount effective to promote re-epithelialization. The anti-connexin peptide may be targeted against a connexin selected from connexin 26, connexin 30, connexin 31.1 and connexin 43. Connexin 43 is presently a preferred target.

In an additional further aspect, the present invention provides a method of promoting wound healing in a subject having a wound that is not healing at the expected rate, including, for example, a delayed healing wound or an incomplete healing wound or a chronic wound, which comprises sustained administration of an gap junction modulating agent, which is effective to regulate epithelial basal cell division and growth. In an additional further aspect, the present invention provides a method of promoting wound healing in a subject having a wound that is not healing at the expected rate, including, for example, a delayed healing wound or an incomplete healing wound or a chronic wound, which comprises sustained administration of an gap junction modulating agent, which is effective to regulate outer layer keratin secretion. In one embodiment, the gap junction modulating agent, e.g., anti-connexin peptide, effective to regulate epithelial basal cell division or growth is an anti-connexin 26 peptide or an anti-connexin 43 peptide or a mixture thereof. According to an alternate embodiment, the gap junction modulating agent, e.g., anti-connexin peptide, is administered to regulate outer layer keratinization is an anti-connexin 31.1 peptide. Repeat applications are included within the invention.

According to another aspect, the present invention provides a method of increasing rate of wound healing or closure in a subject having a wound not healing at the expected rate (including a delayed-healing wound, an incompletely healing wound, and a chronic wound) which comprises administering to the subject an effective amount of a gap junction modulating agents.

In one aspect, the gap junction modulating agent prevents or decreases, in whole or in part, flow of intercellular communication molecules across the gap junction channel. In one aspect, the gap junction modulating agent inhibits intercellular communication by closing the gap junction, in whole or in part. In another aspect, the gap junction modulating agent prevents or decreases, in whole or in part, the formation of a gap junction through hemichannel docking. In another aspect, the gap junction modulating agent induces closure, in whole or in part, of a hemichannel or a gap junction. In yet another aspect the gap junction modulating agent blocks or inhibits, in whole or in part, hemichannel opening. In one aspect, the gap junction modulating agent decreases or prevents, in whole or in part, the opening of a hemichannel or gap junction. In one aspect, the gap junction modulating agent prevents or decreases the activity or function of a hemichannel or a gap junction. Connexin 43 gap junction modulating agents that modulate connexin 43 gap junctions and hemichannels as described herein are preferred.

In one aspect, the invention provides a pharmaceutical composition comprising one or more gap junction modulating agents. Preferably, the pharmaceutical composition further comprises a pharmaceutically acceptable carrier, diluent or excipient. For example, the inventions include pharmaceutical compositions comprising (a) a therapeutically effect amount of a pharmaceutically acceptable connexin 43 gap junction modulating agents and (b) a pharmaceutically acceptable carrier or diluent.

The invention also relates to the use of an gap junction modulating agents in combination with dressings, bandages, matrices and coverings. In one aspect, the invention comprises a synthetic or naturally occurring wound healing matrix comprising an gap junction modulating agent, preferably a connection 43 gap junction modulating agent. In certain embodiments, the gap junction modulating agents is a connexin 43 gap junction modulating agents, for example, an anti-connexin 43 peptide or peptidomimetic.

They also relate to the use of gap junction modulating agents, alone or in combination with dressings, bandages, matrices and coverings, in repeat applications, for example, once per week until healing is complete or as desired.

In one aspect, the gap junction modulating agent is a peptide compound. In one aspect the peptide compound is a peptidomimetic, a peptidomimetic, an antibody or antigen binding fragment thereof. In one aspect, the antigen binding fragment is an F(v), Fab, Fab' or F(ab')₂ anti-connexin 43 antibody fragment. In one aspect, the antibody is a chimeric or humanized antibody. In one aspect, the antibody fragment is a chimeric or humanized antibody fragment. In another aspect, the gap junction modulating agent is a gap junction phosphorylation inducing agent, preferably a connexin 43 gap junction phosphorylation compound.

In one aspect of the invention, the gap junction modulating agent is formulated for topical administration. In one aspect of the invention, the gap junction modulating agent is formulated as a gel. In yet another aspect, the gap junction modulating agent is formulated for slow or extended release. Repeat applications of the gap junction modulating agent are included in the invention. Connexin 43 gap junction modulating agents are preferred.

### DETAILED DESCRIPTION OF THE INVENTION

Wounds that do not heal at expected rates, including slow-healing wounds, delayed-healing wounds, incompletely healing wounds, dehiscent wounds, and chronic wounds often result in infection and can lead to amputation or death. Cell-cell communication through the gap junctions play pivotal roles in wound healing. It has been discovered that use of certain compounds, including those described or referenced herein, can block, inhibit, or alter cell communications, which promotes closure and healing wounds that do not heal at expected rates, including slow-healing wounds, delayed-healing wounds, incompletely healing wounds, dehiscent wounds, and chronic wounds. For all applications, connexin 43 gap junction modulating agents are preferred.

### Definitions

As used herein, a "disorder" is any disorder, disease, or condition that would benefit from an agent that promotes wound healing and/or reduces scar formation. For example, included are wound-associated abnormalities in connection with neuropathic, ischemic, and microvascular pathology; pressure over bony area [tailbone (sacral), hip (trochanteric), buttocks (ischial), or heel of the foot]; reperfusion injury; and conditions associated with valve reflux etiology and related conditions.

As used herein, "subject" refers to any mammals, including humans, domestic and farm animals, and zoo, sports, or pet animals, such as dogs, horses, cats, sheep, pigs, cows, *etc.* The preferred mammal herein is a human, including adults, children, and the elderly.

As used herein, "preventing" means preventing in whole or in part, or ameliorating or controlling.

As used herein, the term "treating" refers to both therapeutic treatment and prophylactic or preventative measures.

As used herein, a "therapeutically effective amount" in reference to the compounds or compositions of the instant invention refers to the amount sufficient to induce a desired biological, pharmaceutical, or therapeutic result. That result can be alleviation of the signs, symptoms, or causes of a disease or disorder or condition, or any other desired alteration of a biological system. In the present invention, the result will involve the promotion and/or improvement of wound healing, including rates of wound healing and closure of wounds, in whole or in part. Other benefits include decreases in swelling, inflammation and/or scar formation, in whole or in part.

As used herein, "simultaneously" is used to mean that the one or more gap junction modulating agents, *e.g*., peptides or blockers, are administered concurrently, whereas the term "in combination" is used to mean they are administered, if not simultaneously or in physical combination, then "sequentially" within a timeframe that they both are available to act therapeutically. Thus, administration "sequentially" may permit one agent to be administered within minutes (for example, 1,2, 3,4, 5, 10, 15, 20, 25, 30) minutes or a matter of hours, days, weeks or months after the other provided that one or more gap junction modulating agents are concurrently present in effective amounts. The time delay between administration of the components will vary depending on the exact nature of the components, the interaction there between, and their respective half-lives.

The terms "peptidomimetic" and "mimetic" include naturally occurring and synthetic chemical compounds that may have substantially the same structural and functional characteristics of protein regions which they mimic. In the case of connexins, these may mimic, for example, the extracellular loops of opposing connexins involved in connexon-connexon docking and cell-cell channel formation, and/or the extracellular loops of hemichannel connexins.

As used herein, the term "peptide analogs" refer to the compounds with properties analogous to those of the template peptide and can be non-peptide drugs. "Peptidomimetics" (also known as peptide mimetics) which include peptide-based compounds, also include such non-peptide based compounds such as peptide analogs. Peptidomimetics that are structurally similar to therapeutically useful peptides can be used to produce an equivalent or enhanced therapeutic or prophylactic effect. Generally, peptidomimetics are structural or functional mimics (e.g. identical or similar) to a paradigm polypeptide (*i.e.,* a polypeptide that has a biological or pharmacological function or activity), but can also have one or more peptide linkages optionally replaced by a linkage selected from the group consisting of, for example, -CH₂NH-, -CH₂S-, -CH₂-CH₂-, - CH=CH- (cis and trans), -COCH₂-, -CH(OH)CH₂-, and -CH₂SO-. The mimetic can be either entirely composed of natural amino acids, synthetic chemical compounds, non-natural analogues of amino acids, or, is a chimeric molecule of partly natural peptide amino acids and partly non-natural analogs of amino acids. The mimetic can also comprise any amount of natural amino acid conservative substitutions as long as such substitutions also do not substantially alter mimetic activity. In the case of connexins, these can mimic, for example, the extracellular loops of opposing connexins involved in connexon-connexon docking and cell-cell channel formation. For example, a mimetic composition can be useful as a gap junction modulating agent if it is capable of down-regulating biological actions or activities of connexons, such as, for example, preventing the docking of connexons to form gap-junction-mediated cell-cell communications, or preventing the opening of connexons to expose the cell cytoplasm to the extracellular millieu. Peptidomimetics encompass those described herein, as well as those as may be known in the art, whether now known or later developed.

In general, the terms "modulator" and "modulation" of gap junction activity, as used herein in its various forms, refers to inhibition in whole or in part of the action or activity of a gap junction or a hemichannel and may function as gap junction modulating agents.

As used herein, the term "protein" refers to any polymer of two or more individual amino acids (whether or not naturally occurring) linked via peptide bonds, as occur when the carboxyl carbon atom of the carboxylic acid group bonded to the alpha-carbon of one amino acid (or amino acid residue) becomes covalently bound to the amino nitrogen atom of the amino group bonded to the alpha-carbon of an adjacent amino acid. These peptide bond linkages, and the atoms comprising them (*i.e*., alpha-carbon atoms, carboxyl carbon atoms (and their substituent oxygen atoms), and amino nitrogen atoms (and their substituent hydrogen atoms)) form the "polypeptide backbone" of the protein. In addition, as used herein, the term "protein" is understood to include the terms "polypeptide" and "peptide" (which, at times, may be used interchangeably herein). Similarly, protein fragments, analogs, derivatives, and variants are may be referred to herein as "proteins," and shall be deemed to be a "protein" unless otherwise indicated. The term "fragment" of a protein refers to a polypeptide comprising fewer than all of the amino acid residues of the protein. A "domain" of a protein is also a fragment, and comprises the amino acid residues of the protein often required to confer activity or function.

The term "wound dressing" refers to a dressing for topical application to a wound and excludes compositions suitable for systemic administration. For example, the one or more gap junction modulating agents, may be dispersed in or on a solid sheet of wound contacting material such as a woven or nonwoven textile material, or may be dispersed in a layer of foam such as polyurethane foam, or in a hydrogel such as a polyurethane hydrogel, a polyacrylate hydrogel, gelatin, carboxymethyl cellulose, pectin, alginate, and/or hyaluronic acid hydrogel, for example in a gel or ointment. In certain embodiments the one or more gap junction modulating agents are dispersed in or on a biodegradable sheet material that provides sustained release of the active ingredients into the wound, for example a sheet of freeze-dried collagen, freeze-dried collagen/alginate mixtures (available under the Registered Trade Mark FIBRACOL from Johnson & Johnson Medical Limited) or freeze-dried collagen/oxidized regenerated cellulose (available under the Registered Trade Mark PROMOGRAN from Johnson & Johnson Medical Limited).

As used herein, "wound promoting matrix" includes for example, synthetic or naturally occurring matrices such as collagen, acellular matrix, crosslinked biological scaffold molecules, tissue based bioengineered structural framework, biomanufactured bioprostheses, and other implanted structures such as for example, vascular grafts suitable for cell infiltration and proliferation useful in the promotion of wound healing. Additional suitable biomatrix material may include chemically modified collagenous tissue to reduce antigenicity and immunogenicity. Other suitable examples include collagen sheets for wound dressings, antigen-free or antigen reduced acellular matrix (Wilson G J et al. (1990) Trans Am Soc Artif Intern 36:340-343) or other biomatrix which have been engineered to reduce the antigenic response to the xenograft material. Other matrices useful in promotion of wound healing may include for example, processed bovine pericardium proteins comprising insoluble collagen and elastin (Courtman DW et al. (1994) J Biomed Mater Res 28:655-666) and other acellular tissue whichh may be useful for providing a natural microenvironment for host cell migration to accelerate tissue regeneration (Malone J M et al. (1984) J Vasc Surg 1:181-91). The invention contemplates a synthetic or natural matrix comprising one or more anti-connexin peptides or peptidomimetics described herein, including anti-connexin 43 peptides and peptidomimetics.

As used herein, the term "wound" includes an injury to any tissue, including for example, delayed or difficult to heal wounds, and chronic wounds. Examples of wounds may include both open and closed wounds. The term "wound" may also include for example, injuries to the skin and subcutaneous tissue initiated in different ways (e.g., pressure sores from extended bed rest and wounds induced by trauma) and with varying characteristics, Wounds may be classified into one of four grades depending on the depth of the wound: i) Grade I: wounds limited to the epithelium; ii) Grade II: wounds extending into the dermis; iii) Grade III: wounds extending into the subcutaneous tissue; and iv) Grade IV (or full-thickness wounds): wounds wherein bones are exposed (e.g., a bony pressure point such as the greater trochanter or the sacrum).

The term "partial thickness wound" refers to wounds that encompass Grades I-III; examples of partial thickness wounds include pressure sores, venous stasis ulcers, and diabetic ulcers. The present invention contemplates treating all wounds of a type that do not heal at expected rates, including, delayed-healing wounds, incompletely healing wounds, and chronic wounds.

By "wound that does not heal at an/the expected rate" is meant an injury to any tissue, including delayed or difficult to heal wounds (including delayed or incompletely healing wounds), and chronic wounds. Examples of wounds that do not heal at the expected rate include ulcers such as diabetic ulcers, diabetic foot ulcers, vascultic ulcers, arterial ulcers, venous ulcers, venous stasis ulcers, burn ulcers, infectious ulcers, trauma-induced ulcers, pressure ulcers, decubitus ulcers, ulcerations associated with pyoderma gangrenosum, and mixed ulcers. Other wounds that do not heal at expected rates include dehiscent wounds.

As used herein, a delayed or difficult to heal wound may include, for example, a wound that is characterized at least in part by 1) a prolonged inflammatory phase, 2) a slow forming extracellular matrix, and/or 3) a decreased rate of epithelialization or closure.

The term "chronic wound" refers to a wound that has not healed. Wounds that do not heal within three months, for example, are considered chronic. Chronic wounds include, for example, pressure ulcers, diabetic ulcers, diabetic foot ulcer, venous ulcers, venous stasis ulcers, arterial ulcers, vasculitic ulcers, burn ulcers, trauma-induced ulcers, infectious ulcers, mixed ulcers, and pyoderma gangrenosum. The chronic wound may be an arterial ulcer which comprises ulcerations resulting from complete or partial arterial blockage. The chronic wound may be a venous or venous stasis ulcer which comprises ulcerations resulting from a malfunction of the venous valve and the associated vascular disease. In certain embodiments a method of treating a chronic wound is provided where the chronic wound is characterized by one or more of the following AHCPR stages of pressure ulceration: stage 1, stage 2, stage 3, and /or stage 4.

As used herein, chronic wound may also include, for example, a wound that is characterized at least in part by 1) a chronic self-perpetuating state of wound inflammation, 2) a deficient and defective wound extracellular matrix, 3) poorly responding (senescent) wound cells (e.g. fibroblasts), 4) limited extracellular matrix production, and/or 5) failure of re-epithelialization due in part to lack of the necessary extracellular matrix orchestration and lack of scaffold for migration. Chronic wounds may also be characterized by 1) prolonged inflammation and proteolytic activity leading to ulcerative lesions, including for example, diabetic, pressure (decubitous), venous, and arterial ulcers; 2) progressive deposition of matrix in the affected area, 3) longer repair times, 4) less wound contraction, 5) slower re-epithelialization, and 6) increased thickness of granulation tissue.

Exemplary chronic wounds may include "pressure ulcer." Exemplary pressure ulcers may include all 4 stages of wound classifications based on AHCPR (Agency for Health Care Policy and Research, U.S. Department of Health and Human Services) guidelines, including for example, Stage 1. A stage I pressure ulcer is an observable pressure related alteration of intact skin whose indicators as compared to the adjacent or opposite area on the body may include changes in one or more of the following: skin temperature (warmth or coolness), tissue consistency (firm or boggy feel) and/or sensation (pain, itching). The ulcer appears as a defined area of persistent redness in lightly pigmented skin, whereas in darker skin tones, the ulcer may appear with persistent red, blue, or purple hues. Stage 1 ulceration may include nonblanchable erythema of intact skin and the heralding lesion of skin ulceration. In individuals with darker skin, discoloration of the skin, warmth, edema, induration, or hardness may also be indicators of stage 1 ulceration. Stage 2: stage 2 ulceration may be characterized by partial thickness skin loss involving epidermis, dermis, or both. The ulcer is superficial and presents clinically as an abrasion, blister, or shallow crater. Stage 3: stage 3 ulceration may be characterized by full thickness skin loss involving damage to or necrosis of subcutaneous tissue that may extend down to, but not through, underlying fascia. The ulcer presents clinically as a deep crater with or without undermining of adjacent tissue. Stage 4: stage 4 ulceration may be characterized by full thickness skin loss with extensive destruction, tissue necrosis, or damage to muscle, bone, or supporting structures (*e.g*., tendon, joint capsule). In certain embodiments a method of treating a chronic wound is provided where the chronic wound is characterized by one or more of the following AHCPR stages of pressure ulceration: stage 1, stage 2, stage 3, and/or stage 4.

Exemplary chronic wounds may include "decubitus ulcers." Exemplary decubitus ulcers may arise as a result of prolonged and unrelieved pressure over a bony prominence that leads to ischemia. The wound tends to occur in patients who are unable to reposition themselves to off-load weight, such as paralyzed, unconscious, or severely debilitated persons. As defined by the U.S. Department of Health and Human Services, the major preventive measures include identification of high-risk patients; frequent assessment; and prophylactic measures such as scheduled repositioning, appropriate pressure-relief bedding, moisture barriers, and adequate nutritional status. Treatment options may include for example, pressure relief, surgical and enzymatic debridement, moist wound care, and control of the bacterial load. In certain embodiments a method of treating a chronic wound is provided wherein the chronic wound is characterized by decubitus ulcer or ulceration which results from prolonged, unrelieved pressure over a bony prominence that leads to ischemia. In certain embodiments a method of treating a chronic wound is provided wherein the chronic wound is characterized by decubitus ulcer or ulceration which results from prolonged, unrelieved pressure over a bony prominence that leads to ischemia.

Exemplary chronic wounds may include "arterial ulcers." Chronic arterial ulcers are generally understood to be ulcerations that accompany arteriosclerotic and hypertensive cardiovascular disease. They are painful, sharply marginated, and often found on the lateral lower extremities and toes. Arterial ulcers may include those ulcers characterized by complete or partial arterial blockage which may lead to tissue necrosis and/or ulceration. Signs of arterial ulcer may include, for example, pulselessness of the extremity; painful ulceration; small, punctate ulcers that are usually well circumscribed; cool or cold skin; delayed capillary return time (briefly push on the end of the toe and release, normal color should return to the toe in about 3 seconds or less); atrophic appearing skin (for example, shiny, thin, dry); and loss of digital and pedal hair.

Exemplary chronic wounds may include "venous ulcers." Exemplary venous ulcers may include the most common type of ulcer affecting the lower extremities and may be characterized by malfunction of the venous valve. The normal vein has valves that prevent the backflow of blood. When these valves become incompetent, the backflow of venous blood causes venous congestion. Hemoglobin from the red blood cells escapes and leaks into the extravascular space, causing the brownish discoloration commonly noted. It has been shown that the transcutaneous oxygen pressure of the skin surrounding a venous ulcer is decreased, suggesting that there are forces obstructing the normal vascularity of the area. Lymphatic drainage and flow also plays a role in these ulcers. The venous ulcer may appear near the medial malleolus and usually occurs in combination with an edematous and indurated lower extremity; it may be shallow, not too painful and may present with a weeping discharge from the affected site. In certain embodiments a method of treating a chronic wound is provided wherein the chronic wound is characterized by arterial ulcers or ulcerations due to complete or partial arterial blockage.

Exemplary chronic wounds may include "venous stasis ulcers." Stasis ulcers are lesions associated with venous insufficiency are more commonly present over the medial malleolus, usually with pitting edema, varicosities, mottled pigmentation, erythema, and nonpalpable petechiae and purpura. The stasis dermatitis and ulcers are generally pruritic rather than painful. Exemplary venous stasis ulcers may be characterized by chronic passive venous congestion of the lower extremities results in local hypoxia. One possible mechanism of pathogenesis of these wounds includes the impediment of oxygen diffusion into the tissue across thick perivascular fibrin cuffs. Another mechanism is that macromolecules leaking into the perivascular tissue trap growth factors needed for the maintenance of skin integrity. Additionally, the flow of large white blood cells slows due to venous congestion, occluding capillaries, becoming activated, and damaging the vascular endothelium to predispose to ulcer formation. In certain embodiments a method of treating a chronic wound is provided wherein the chronic wound is characterized by venous ulcers or ulcerations due to malfunction of the venous valve and the associated vascular disease. In certain embodiments a method of treating a chronic wound is provided wherein the chronic wound is characterized by venous stasis ulcers or ulcerations due to chronic passive venous congestion of the lower extremities and/or the resulting local hypoxia.

Exemplary chronic wounds may include "diabetic ulcers." Diabetic patients are prone to ulcerations, including foot ulcerations, due to both neurologic and vascular complications. Peripheral neuropathy can cause altered or complete loss of sensation in the foot and/or leg. Diabetic patients with advanced neuropathy loose all ability for sharp-dull discrimination. Any cuts or trauma to the foot may go completely unnoticed for days or weeks in a patient with neuropathy. It is not uncommon to have a patient with neuropathy notice that the ulcer "just appeared" when, in fact, the ulcer has been present for quite some time. For patients of neuropathy, strict glucose control has been shown to slow the progression of the disease. Charcot foot deformity may also occur as a result of decreased sensation. People with "normal" feeling in their feet have the ability to sense automatically when too much pressure is being placed on an area of the foot. Once identified, our bodies instinctively, shift position to relieve this stress. A patient with advanced neuropathy looses this ability to sense the sustained pressure insult, as a result, tissue ischemia and necrosis may occur leading to for example, plantar ulcerations. Additionally, microfractures in the bones of the foot, if unnoticed and untreated, may result in disfigurement, chronic swelling and additional bony prominences. Microvascular disease is one of the significant complications for diabetics which may also lead to ulcerations. In certain embodiments a method of treating a chronic wound is provided wherein the chronic wound is characterized by diabetic foot ulcers and/or ulcerations due to both neurologic and vascular complications of diabetes.

Exemplary chronic wounds can include "traumatic ulcers." Formation of exemplary traumatic ulcers may occur as a result of traumatic injuries to the body. These injuries include, for example, compromises to the arterial, venous or lymphatic systems; changes to the bony architecture of the skeleton; loss of tissue layers-epidermis, dermis, subcutaneous soft tissue, muscle or bone; damage to body parts or organs and loss of body parts or organs. In certain embodiments, a method of treating a chronic wound is provided wherein the chronic wound is characterized by ulcerations associated with traumatic injuries to the body.

Exemplary chronic wounds can include "burn ulcers" including for example, ulceration that occur as a result of a burn injury, including 1st degree burn (i.e. superficial, reddened area of skin); 2nd degree burn (a blistered injury site which may heal spontaneously after the blister fluid has bee removed); 3rd degree burn (bum through the entire skin and usually require surgical intervention for wound healing); scalding (may occur from scalding hot water, grease or radiator fluid); thermal (may occur from flames, usually deep bums); chemical (may come from acid and alkali, usually deep bums); electrical (either low voltage around a house or high voltage at work); explosion flash (usually superficial injuries); and contact burns (usually deep and may occur from muffler tail pipes, hot irons and stoves). In certain embodiments, a method of treating a chronic wound is provided wherein the chronic wound is characterized by ulcerations associated with burn injuries to the body.

Exemplary chronic wounds can include "vasculitic ulcers." Vasculitic ulcers also occur on the lower extremities and are painful, sharply marginated lesions, which may have associated palpable purpuras and hemorrhagic bullae. The collagen diseases, septicemias, and a variety of hematological disorders (e.g., thrombocytopenia, dysproteinemia) may be the cause of this severe, acute condition.

Exemplary chronic wounds can include pyoderma gangrenosum. Pyoderma gangrenosum occurs as single or multiple, very tender ulcers of the lower legs. A deep red to purple, undermined border surrounds the purulent central defect. Biopsy typically fails to reveal a vasculitis. In half the patients it is associated with a systemic disease such as ulcerative colitis, regional ileitis, or leukemia. In certain embodiments, a method of treating a chronic wound is provided wherein the chronic wound is characterized by ulcerations associated with pyoderma gangrenosum.

Exemplary chronic wounds can include ocular ulcers, including corneal ulcers or indulent ulcers. Also included are persistent epithelial defects of the eye. These may occur in humans and also in sport animals (such as horses) and pet animals (including dogs).

Exemplary chronic wounds can include infectious ulcers. Infectious ulcers follow direct innoculation with a variety of organisms and may be associated with significant regional adenopathy. Mycobacteria infection, anthrax, diphtheria, blastomyosis, sporotrichosis, tularemia, and cat-scratch fever are examples. The genital ulcers of primary syphilis are typically nontender with a clean, firm base. Those of chancroid and granuloma inguinale tend to be ragged, dirty, and more extravagant lesions. In certain embodiments, a method of treating a chronic wound is provided wherein the chronic wound is characterized by ulcerations associated with infection.

As used herein, the term "dehiscent wound" refers to a wound, usually a surgical wound, which has ruptured or split open. In certain embodiments, a method of treating a wound that does not heal at the expected rate is provided wherein the wound is characterized by dehiscence.

### Gap Junction Modulating Agents

Certain agents described herein are capable of modulating or affecting the transport of molecules into and out of cells (e.g. blocking or inhibiting). Thus certain gap junction modulating agents described herein modulate cellular communication (e.g. cell to cell). Certain gap junction modulating agents modulate or affect transmission of molecules between the cell cytoplasm and the periplasmic or extracellular space. Such agents are generally targeted to hemichannels (also called connexons), which may be independently involved in the exchange of small molecules between the cell cytoplasm and an extracellular space or tissue. Thus, a compound provided herein may directly or indirectly reduce coupling between cells (via gap junctions) or between a cell and an extracellular space or tissue (via hemichannels), and the modulation of transport of molecules from a cell into an extracellular space is within the scope of certain compounds and embodiments of the invention.

Any molecule that is capable of eliciting a desired inhibition of the passage (e.g. transport) of molecules through a gap junction or hemichannel may be used in embodiments of the invention. Compounds that modulate the passage of molecules through a gap junction or hemichannel are also provided in particular embodiments (*e.g*., those that modulate the passage of molecules from the cytoplasm of a cell into an extracellular space). Such compounds may modulate the passage of molecules through a gap junction or hemichannel with or without gap junction uncoupling. Such compounds include, for example, binding proteins, polypeptides, and other organic compound that can, for example, block the function or activity of a gap junction or a hemichannel in whole or in part.

Certain gap junction inhibitors are listed in Evans, W.H. and Boitano, S. Biochem. Soc. Trans. 29: 606-612 (2001). Peptide inhibitors (including peptidomimetics) of gap junctions and hemichannels have been reported. See for example Berthoud, V.M. et al., Am J. Physiol. Lung Cell Mol. Physiol. 279: L619-L622 (2000); De Vriese et al., Kidney Int. 61:177-185 (2001); Boitano S. and Evans W. Am J Physiol Lung Cell Mol Physiol 279: L623-L630 (2000).

As used herein, "gap junction modulating agent" may broadly include those agents or compounds that prevent, decrease or modulate, in whole or in part, the activity, function, or formation of a hemichannel or a gap junction. In certain embodiments, a gap junction modulating agent prevents or decreases, in whole or in part, the function of a hemichannel or a gap junction. In certain embodiments, a gap junction modulating agent induces closure, in whole or in part, of a hemichannel or a gap junction. In other embodiments, a gap junction modulating agent blocks, in whole or in part, a hemichannel or a gap junction. In certain embodiments, a gap junction modulating agent decreases or prevents, in whole or in part, the opening of a hemichannel or gap junction. In certain embodiments, said blocking or closure of a gap junction or hemichannel by a gap junction modulating agent can reduce or inhibit extracellular hemichannel communication by preventing or decreasing the flow of small molecules through an open channel to and from an extracellular or periplasmic space. Peptidomimetics, and gap junction phosphorylation compounds that block hemichannel and/or gap junction opening are presently preferred.

In certain embodiments, a gap junction modulating agent prevents, decreases or alters the activity or function of a hemichannel or a gap junction. As used herein, modification of the gap junction activity or function may include the closing of gap junctions, closing of hemichannels, and/or passage of molecules or ions through gap junctions and/or hemichannels.

In certain another aspect, gap junction modulating agent may include, for example, aliphatic alcohols; octanol; heptanol; anaesthetics (*e.g*. halothane), ethrane, fluothane, propofol and thiopental; anandamide; arylaminobenzoate (FFA: flufenamic acid and similar derivatives that are lipophilic); carbenoxolone; Chalcone: (2',5'-dihydroxychalcone); CHFs (Chlorohydroxyfuranones); CMCF (3-chloro-4-(chloromethyl)-5-hydroxy-2(5H)-furanone); dexamethasone; doxorubicin (and other anthraquinone derivatives); eicosanoid thromboxane A(2) (TXA(2)) mimetics; NO (nitric oxide); Fatty acids (e.g. arachidonic acid, oleic acid and lipoxygenase metabolites; Fenamates (flufenamic (FFA), niflumic (NFA) and meclofenamic acids (MFA)); Genistein; glycyrrhetinic acid (GA):18*a*-glycyrrhetinic acid and 18-beta - glycyrrhetinic acid, and derivatives thereof; lindane; lysophosphatidic acid; mefloquine; menadione; 2-Methyl-1,4-naphthoquinone, vitamin K(3); nafenopin; okadaic acid; oleamide; oleic acid; PH, gating by intracellular acidification; *e.g.* acidifying agents; polyunsaturated fatty acids; fatty acid GJIC inhibitors (e.g. oleic and arachidonic acids); quinidine; quinine; all trans-retinoic acid; and tamoxifen.

Exemplary gap junction modulating agents may include, without limitation, polypeptides (e.g. peptiditomimetics, antibodies, binding fragments thereof, and synthetic constructs), and other gap junction blocking agents, and gap junction protein phosphorylating agents. Exemplary compounds used for closing gap junctions (e.g. phosphorylating connexin 43 tyrosine residue) have been reported in U.S. Pat. No. 7,153,822 to Jensen et al.*,* U.S. Pat. No. 7,250,397, and assorted patent publications. Exemplary peptides and peptidomimetics are reported in Green et al., WO2006134494. See also Gourdie *et al.,* see W02006069181, and Griffith, see WO2003032964.

As used herein, "gap junction phosphorylating agent" may include those agents or compounds capable of inducing phosphorylation on connexin amino acid residues in order to induce gap junction or hemichannel closure and, thus, gap junction modulation. Exemplary sites of phosphorylation include one or more of a tyrosine, serine or threonine residues on the connexin protein. In certain embodiments, modulation of phosphorylation may occur on one or more residues on one or more connexin proteins. Exemplary gap junction phosphorylating agents are well known in the art and may include, for example, c-Src tyrosine kinase or other G protein-coupled receptor agonists. *See* Giepmans B (2001) J. Biol. Chem., Vol. 276, Issue 11, 8544-8549. In one embodiment, modulation of phosphorylation on one or more of these residues impacts hemichannel function, particularly by closing the hemichannel. In another embodiment, modulation of phosphorylation on one or more of these residues impacts gap junction function, particularly by closing the gap junction. Gap junction phosphorylating agents that target the closure of connexin 43 gap junctions and hemichannels are preferred.

Polypeptide compounds, including binding proteins (e.g. antibodies, antibody fragments, and the like), peptides, peptidomimetics, and peptidomimetics, are suitable modulators of gap junctions.

Binding proteins include, for example, monoclonal antibodies, polyclonal antibodies, antibody fragments (including, for example, Fab, F(ab')₂ and Fv fragments; single chain antibodies; single chain Fvs; and single chain binding molecules such as those comprising, for example, a binding domain, hinge, CH2 and CH3 domains, recombinant antibodies and antibody fragments which are capable of binding an antigenic determinant (*i.e.,* that portion of a molecule, generally referred to as an epitope) that makes contact with a particular antibody or other binding molecule. These binding proteins, including antibodies, antibody fragments, and so on, may be chimeric or humanized or otherwise made to be less immunogenic in the subject to whom they are to be administered, and may be synthesized, produced recombinantly, or produced in expression libraries. Any binding protein known in the art or later discovered is envisioned, such as those referenced herein and/or described in greater detail in the art. For example, binding proteins include not only antibodies, and the like, but also ligands, receptors, peptidomimetics, or other binding fragments or molecules (for example, produced by phage display) that bind to a target (*e.g*. connexin, connexon, gap junctions, or associated molecules).

Binding molecules will generally have a desired specificity, including but not limited to binding specificity, and desired affinity. Affinity, for example, may be a Kₐ of greater than or equal to about 10⁴ M⁻¹, greater than or equal to about 10⁶ M⁻¹, greater than or equal to about 10⁷ M⁻¹, greater than or equal to about 10⁸ M⁻¹. Affinities of even greater than about 10⁸ M⁻¹ are suitable, such as affinities equal to or greater than about 10⁹ M⁻¹, about 10¹⁰ M⁻¹, about 10¹¹ M⁻¹, and about 10¹² M⁻¹. Affinities of binding proteins according to the present invention can be readily determined using conventional techniques, for example those described by Scatchard et al., (1949) Ann. N.Y. Acad. Sci. 51: 660.

The invention includes use of peptides (including peptidomimetic and peptidomimetics) for modulation of gap junctions and hemichannels. By using data obtained from hydropathy plots, it has been proposed that a connexin contains four-transmembrane-spanning regions and two short extra-cellular loops. The positioning of the first and second extracellular regions of connexin was further characterized by the reported production of antipeptide antibodies used for immunolocalization of the corresponding epitopes on split gap junctions. Goodenough D.A. (1988) J Cell Biol 107: 1817-1824; Meyer R.A. (1992) J Cell Biol 119: 179-189*.*

Peptides or variants thereof, can be synthesized *in vitro, e.g.,* by the solid phase peptide synthetic method or by enzyme-catalyzed peptide synthesis or with the aid of recombinant DNA technology. Solid phase peptide synthetic method is an established and widely used method, which is described in references such as the following: Stewart et al., (1969) Solid Phase Peptide Synthesis, W. H. Freeman Co., San Francisco; Merrifield, (1963) J. Am. Chem. Soc. 85 2149; Meienhofer in "Hormonal Proteins and Peptides," ed.; C.H. Li, Vol.2 (Academic Press, 1973), pp.48-267; and Bavaay and Merrifield, "The Peptides," eds. E. Gross and F. Meienhofer, Vol.2 (Academic Press, 1980) pp.3-285. These peptides can be further purified by fractionation on immunoaffinity or ion-exchange columns; ethanol precipitation; reverse phase HPLC; chromatography on silica or on an anion-exchange resin such as DEAE; chromatofocusing; SDS-PAGE; ammonium sulfate precipitation; gel filtration using, for example, Sephadex G-75; ligand affinity chromatography; or crystallization or precipitation from non-polar solvent or nonpolar/polar solvent mixtures. Purification by crystallization or precipitation is preferred.

The extracellular domains of a hemichannel contributed by two adjacent cells "dock" with each other to form complete gap junction channels. Reagents that interfere with the interactions of these extracellular domains can impair cell-to-cell communication, or with hemichannel opening to the extracellular environment.

Gap junction modulating agents include peptides comprising an amino acid sequence corresponding to a transmembrane region (*e.g.* 1^{st} to 4^{th}) of a connexin (*e.g.* connexin 45, 43, 26, 30, 31.1, and 37). Gap junction modulating agents including a peptide comprising an amino acid sequence corresponding to a portion of a transmembrane region of a connexin 43 are preferred for use in the present inventions.

Gap junction modulating agents may comprise a peptide comprising an amino acid sequence corresponding to a portion of a transmembrane region of a connexin 45. Gap junction modulating agents include a peptide having an amino acid sequence that comprises about 5 to 20 contiguous amino acids of SEQ.ID.NO:1, a peptide having an amino acid sequence that comprises about 8 to 15 contiguous amino acids of SEQ.ID.NO: 1, or a peptide having an amino acid sequence that comprises about 11 to 13 contiguous amino acids of SEQ.ID.NO:1. Other embodiments are directed to an gap junction modulating compound that is a peptide having an amino acid sequence that comprises at least about 5, at least about 6, at least about 7, at least about 8, at least about 9, at least about 10, at least about 11, at least about 12, at least about 13, at least about 14, at least about 15, at least about 20, at least about 25, or at least about 30 contiguous amino acids of SEQ.ID.NO:1. In certain gap junction modulating compounds provided herein, the extracellular domains of connexin 45 corresponding to the amino acids at positions 46-75 and 199-228 of SEQ ID NO: 1 may be used to develop the particular peptide sequences. Certain peptides described herein have an amino acid sequence corresponding to the regions at positions 46-75 and 199-228 of SEQ.ID.NO: 1. The peptides need not have an amino acid sequence identical to those portions of SEQ.ID.NO: 1, and conservative amino acid changes may be made such that the peptides retain binding activity or functional activity. Alternatively, the peptide may target regions of the connexin protein other than the extracellular domains (*e.g*. the portions of SEQ.ID.NO:1 not corresponding to positions 46-75 and 199-228).

Also, suitable gap junction modulating agents can include a peptide comprising an amino acid sequence corresponding to a portion of a transmembrane region of a connexin 43. Gap junction modulating agents include peptides having an amino acid sequence that comprises about 5 to 20 contiguous amino acids of SEQ.ID.NO:2, peptides having an amino acid sequence that comprises about 8 to 15 contiguous amino acids of SEQ.ID.NO:2, or peptides having an amino acid sequence that comprises about 11 to 13 contiguous amino acids of SEQ.ID.NO:2. Other gap junction modulating agents include a peptide having an amino acid sequence that comprises at least about 5, at least about 6, at least about 7, at least about 8, at least about 9, at least about 10, at least about 11, at least about 12, at least about 13, at least about 14, at least about 15, at least about 20, at least about 25, or at least about 30 contiguous amino acids of SEQ.ID.NO:2. Other gap junction modulating agents comprise the extracellular domains of connexin 43 corresponding to the amino acids at positions 37-76 and 178-208 of SEQ.ID.NO:2. Gap junction modulating agents include peptides described herein which have an amino acid sequence corresponding to the regions at positions 37-76 and 178-208 of SEQ.ID.NO:2. The peptides need not have an amino acid sequence identical to those portions of SEQ.ID.NO:2, and conservative amino acid changes may be made such that the peptides retain binding activity or functional activity. Alternatively, peptides may target regions of the connexin protein other than the extracellular domains (e.g. the portions of SEQ.ID.NO:2 not corresponding to positions 37-76 and 178-208).
Other gap junction modulating agents include carboxy terminal polypeptides.

Connexin 45 (SEQ.ID.NO:1)

Met Ser Trp Ser Phe Leu Thr Arg Leu Leu Glu Glu Ile His Asn His
1               5                   10                  15

Ser Thr Phe Val Gly Lys Ile Trp Leu Thr Val Leu Ile Val Phe Arg
            20                  25                  30

Ile Val Leu Thr Ala Val Gly Gly Glu Ser Ile Tyr Tyr Asp Glu Gln
        35                  40                  45

Ser Lys Phe Val Cys Asn Thr Glu Gln Pro Gly Cys Glu Asn Val Cys
    50                  55                  60

Tyr Asp Ala Phe Ala Pro Leu Ser His Val Arg Phe Trp Val Phe Gln
65                  70                  75                  80

Ile Ile Leu Val Ala Thr Pro Ser Val Met Tyr Leu Gly Tyr Ala Ile
                85                  90                  95

His Lys Ile Ala Lys Met Glu His Gly Glu Ala Asp Lys Lys Ala Ala
            100                 105                 110

Arg Ser Lys Pro Tyr Ala Met Arg Trp Lys Gln His Arg Ala Leu Glu
        115                 120                 125

Glu Thr Glu Glu Asp Asn Glu Glu Asp Pro Met Met Tyr Pro Glu Met
    130                 135                 140

Glu Leu Glu Ser Asp Lys Glu Asn Lys Glu Gln Ser Gln Pro Lys Pro
145                 150                 155                 160

Lys His Asp Gly Arg Arg Arg Ile Arg Glu Asp Gly Leu Met Lys Ile
                165                 170                 175

Tyr Val Leu Gln Leu Leu Ala Arg Thr Val Phe Glu Val Gly Phe Leu
            180                 185                 190

Ile Gly Gln Tyr Phe Leu Tyr Gly Phe Gln Val His Pro Phe Tyr Val
        195                 200                 205

Cys Ser Arg Leu Pro Cys Pro His Lys Ile Asp Cys Phe Ile Ser Arg
    210                 215                 220

Pro Thr Glu Lys Thr Ile Phe Leu Leu Ile Met Tyr Gly Val Thr Gly
225                 230                 235                 240

Leu Cys Leu Leu Leu Asn Ile Trp Glu Met Leu His Leu Gly Phe Gly
                245                 250                 255

Thr Ile Arg Asp Ser Leu Asn Ser Lys Arg Arg Glu Leu Glu Asp Pro
            260                 265                 270

Gly Ala Tyr Asn Tyr Pro Phe Thr Trp Asn Thr Pro Ser Ala Pro Pro
        275                 280                 285

Gly Tyr Asn Ile Ala Val Lys Pro Asp Gln Ile Gln Tyr Thr Glu Leu
    290                 295                 300

Ser Asn Ala Lys Ile Ala Tyr Lys Gln Asn Lys Ala Asn Thr Ala Gln
305                 310                 315                 320

Glu Gln Gln Tyr Gly Ser His Glu Glu Asn Leu Pro Ala Asp Leu Glu
                325                 330                 335

Ala Leu Gln Arg Glu Ile Arg Met Ala Gln Glu Arg Leu Asp Leu Ala
            340                 345                 350

Val Gln Ala Tyr Ser His Gln Asn Asn Pro His Gly Pro Arg Glu Lys
        355                 360                 365

Lys Ala Lys Val Gly Ser Lys Ala Gly Ser Asn Lys Ser Thr Ala Ser
    370                 375                 380

Ser Lys Ser Gly Asp Gly Lys Asn Ser Val Trp Ile
385                 390                 395

Connexin 43 (SEQ.ID.NO:2)


1               5                   10                  15


            20                  25                  30


        35                  40                  45


    50                  55                  60


65                  70                  75                  80


                85                  90                  95


            100                 105                 110


        115                 120                 125


    130                 135                 140


145                 150                 155                 160


                165                 170                 175



180                 185                 190


    195                 200                 205


    210                 215                 220


225                 230                 235                 240


                245                 250                 255


            260                 265                 270


        275                 280                 285


    290                 295                 300


305                 310                 315                 320


                325                 330                 335


            340                 345                 350


        355                 360                 365


    370                 375                 380

The gap junction modulating peptides may comprise sequences corresponding to a portion of the connexin extracellular domains with conservative amino acid substitutions such that peptides are functionally active gap junction modulating compounds. Exemplary conservative amino acid substitutions include for example the substitution of a nonpolar amino acid with another nonpolar amino acid, the substitution of an aromatic amino acid with another aromatic amino acid, the substitution of an aliphatic amino acid with another aliphatic amino acid, the substitution of a polar amino acid with another polar amino acid, the substitution of an acidic amino acid with another acidic amino acid, the substitution of a basic amino acid with another basic amino acid, and the substitution of an ionizable amino acid with another ionizable amino acid.

Exemplary peptides targeted to connexin 43 are shown below in Table 1. M1, 2, 3 and 4 refer to the 1^{st} to 4^{th} transmembrane regions of the connexin 43 protein respectively. E2 refer to the first and second extracellular loops respectively.

**Table 1. Peptidic Inhibitors of Intercellular Communication (cx43)**

| | | |
|---|---|---|
| FEVAFLLIQWI | M3 & E2 | (SEQ.ID.NO:3) |
| LLIQWYIGFSL | E2 | (SEQ.ID.NO:4) |
| SLSAVYTCKRDPCPHQ | E2 | (SEQ.ID.NO:5) |
| VDCFLSRPTEKT | E2 | (SEQ.ID.NO:6) |
| SRPTEKTIFII | E2 & M4 | (SEQ.ID.NO:7) |
| LGTAVESAWGDEQ | M1 & E1 | (SEQ.ID.NO:8) |
| QSAFRCNTQQPG | E1 | (SEQ.ID.NO:9) |
| QQPGCENVCYDK | E1 | (SEQ.ID.NO:10) |
| VCYDKSFPISHVR | E1 | (SEQ.ID.NO:11) |

Table 2 provides additional exemplary connexin peptides used in inhibiting hemichannel or gap junction function. In other embodiments, conservative amino acid changes are made to the peptides or fragments thereof.

**Table 2. Additional Peptidic Inhibitors of Intercellular Communication (cx32, cx43)**

| Connexin | Location | | AA's and Sequence |
|---|---|---|---|
| Cx32 | E1 39-7 | AAESVWGDEIKSSFICNTLQPGCNSVCYDHFFPISHVR | (SEQ.ID.NO: 12) |
| Cx32 | E1 41-52 | ESVWGDEKSSFI | (SEQ.ID.NO: 13) |
| Cx32 | E1 52-63 | ICNTLQPGCNSV | (SEQ.ID.NO: 14) |
| Cx32 | E1 62-73 | SVCYDHFFPISH | (SEQ.ID.NO: 15) |
| Cx32 | E2 64-188 | RLVKCEAFPCPNTVDCFVSRPTEKT | (SEQ.ID.NO: 16) |
| Cx32 | E2 166-177 | VKCEAFPCPNTV | (SEQ.ID.NO: 17) |
| Cx32 | E2 177-188 | VDCFVSRPTEKT | (SEQ.ID.NO: 18) |
| Cx32 | E1 63-75 | VCYDHFFPISHVR | (SEQ.ID.NO: 19) |
| Cx32 | E1 45-59 | VWGDEKSSFICNTLQPGY | (SEQ.ID.NO: 20) |
| Cx32 | E1 46-59 | DEKSSFICNTLQPGY | (SEQ.ID.NO: 21) |
| Cx32 | E2 182-192 | SRPTEKTVFTV | (SEQ.ID.NO: 22) |
| Cx32/Cx43 | E2 182-188/ 201-207 | SRPTEKT | (SEQ.ID.NO: 23) |
| Cx32 | E1 52-63 | ICNTLQPGCNSV | (SEQ.ID.NO: 24) |
| Cx40 | E2 177-192 | FLDTLHVCRRSPCPHP | (SEQ.ID.NO: 25) |
| Cx43 | E2 188-205 | KRDPCHQVDCFLSRPTEK | (SEQ.ID.NO: 26) |

Table 3 provides the extracellular loops for connexin family members which are used to develop peptide inhibitors described herein. The peptides and provided in Table 4, and fragments thereof, are used as peptide inhibitors in certain non-limiting embodiments. In other non-limiting embodiments, peptides comprising from about 8 to about 15, of from about 11 to about 13 amino contiguous amino acids of the peptides in this Table are peptide inhibitors of the invention. Conservative amino acid changes may be made to the peptides or fragments thereof. *See* Boitano S. and Evans W. *Am J Physiol Lung Cell Mol Physiol* 279: L623-L630 (2000).

**Table 3. Extracellular loops for various connexin family members**

| **E1** | | |
|---|---|---|
| huCx26 | KEVWGDEQADFVCNTLQPGCKNVCYDHYFPISHIR | (SEQ.ID.NO: 27) |
| huCx30 | QEVWGDEQEDFVCNTLQPGCKNVCYDHFFPVSHIR | (SEQ.ID.NO: 28) |
| huCx30.3 | EEVWDDEQKDFVCNTKQPGCPNVCYDEFFPVSHVR | (SEQ.ID.NO: 29) |
| huCx31 | ERVWGDEQKDFDCNTKQPGCTNVCYDNYFPISNIR | (SEQ.ID.NO: 30) |
| huCx31.1 | ERVWSDDHKDFDCNTRQPGCSNVCFDEFFPVSHVR | (SEQ.ID.NO: 31) |
| huCx32 | ESVWGDEKSSFICNTLQPGCNSVCYDQFFPISHVR | (SEQ.ID.NO: 32) |
| huCx36 | ESVWGDEQSDFECNTAQPGCTNVCYDQAFPISHIR | (SEQ.ID.NO: 33) |
| huCx37 | ESVWGDEQSDFECNTAQPGCTNVCYDQAFPISHIR | (SEQ.ID.NO: 34) |
| huCx40.1 | RPVYQDEQERFVCNTLQPGCANVCYDVFS PVSHLR | (SEQ.ID.NO: 35) |
| huCx43 | ESAWGDEQSAFRCNTQQPGCENVCYDKSFPISHVR | (SEQ.ID.NO: 36) |
| huCx46 | EDVWGDEQSDFTCNTQQPGCBNVCYBRAFPISHIR | (SEQ.ID.NO: 37) |
| huCx46.6 | EAIYSDEQAKFTCNTRQPGCDNVCYDAFAPLSHVR | (SEQ.ID.NO: 38) |
| huCx40 | ESSWGDEQADFRCDTIQPGCQNVCTDQAFPISHIR | (SEQ.ID.NO: 39) |
| huCx45 | GESIYYDEQSKFVCNTEQPGCENVCYDAFAPLSHVR | (SEQ.ID.N0: 40) |

| **E2** | | |
|---|---|---|
| huCx26 | MYVFYVMYDGFSMQRLVKCNAWPCPNTVDCFVSRPTEKT | (SEQ.ID.NO: 41) |
| huCx30 | MYVFYFLYNGYHLPWVLKCGIDPCPNLVDCFISRPTEKT | (SEQ.ID.NO: 42) |
| huCx30.3 | LYIFHRLYKDYDMPRVVACSVEPCPHTVDCYISRPTEKK | (SEQ.ID.NO: 43) |
| huCx31 | LYLLHTLWHGFNMPRLVQCANVAPCPNIVDCYIARPTEKK | (SEQ.ID.NO: 44) |
| huCx31.1 | LYVFHSFYPKYILPPVVKCHADPCPNIVDCFISKPSEKN | (SEQ.ID.NO: 45) |
| huCx32 | MYVFYLLYPGYAMVRLVKCDVYPCPNTVDCFVSRPTEKT | (SEQ.ID.NO: 46) |
| huCx36 | LYGWTMEPVFVCQRAPCPYLVDCFVSRPTEKT | (SEQ.ID.NO: 47) |
| huCx37 | LYGWTMEPVFVCQRAPCPYLVDCFVSRPTEKT | (SEQ.ID.NO: 48) |
| huCx40.1 | GALHYFLFGFLAPKKFPCTRPPCTGVVDCYVSRPTSKS | (SEQ.ID.NO: 49) |
| huCx43 | LLIQWYIYGFSLSAVYTCKRDPCPHQVDCFLSRPTEKT | (SEQ.ID.NO: 50) |
| huCx46 | IAGQYFLYGFELKPLYRCDRWPCPNTVDCFISRPTEKT | (SEQ.ID.NO: 51) |
| huCx46.6 | LVGQYLLYGFEVRPFFPCSRQPCPHVVDCFVSRPTEKT | (SEQ.ID.NO: 52) |
| hucx40 | IVGQYFIYGIFLTTLHVCRRSPCPHPVNCYVSRPTEKN | (SEQ.ID.NO: 53) |
| huCx45 | LIGQYFLYGFQVHPFYVCSRLPCHPKIDCFISRPTEKT | (SEQ.ID.NO: 54) |

Sequences of the E2 domain of different connexin isotypes are shown in Table 3. Note that last 4 amino acids of peptide SEQ.ID.NO:7 are part of the fourth membrane domain.

Table 4 provides the extracellular domain for connexin family members which may be used to develop peptide gap junction modulating agents. The peptides and provided in Table 4, and fragments thereof, may also be used as peptide gap junction modulating agents. Such peptides may comprise from about 8 to about 15, or from about 11 to about 13 amino contiguous amino acids of the peptide sequence in this Table 4. Conservative amino acid changes may be made to the peptides or fragments thereof.

**Table 4. Extracellular domains**

| | | |
|---|---|---|
| Peptide | VDCFLSRPTEKT | (SEQ.ID.NO: 6) |
| Peptide | SRPTEKTIFII | (SEQ.ID.NO: 7) |
| huCx43 | LLIQWYIYGFSLSAVYTCKRDPCPHQVDCFLSRPTEKTIFII | (SEQ.ID.NO: 55) |
| huCx26 | MYVFYVMYDGFSMQRLVKCNAWPCPNTVDCFVSRPTEKTVFTV | (SEQ.ID.NO: 56) |
| huCx30 | YVFYFLYNGYHLPWVLKCGIDPCPNLVDCFISRPTEKTVFTI | (SEQ.ID.NO: 57) |
| huCx30.3 | LYIFHRLYKDYDMPRVVACSVEPCPHTVDCYISRPTEKKVFTY | (SEQ.ID.NO: 58) |
| huCx31 | LYLLHTLWHGFNMPRLVQCANVAPCPNIVDCYIARPTEKKTY | (SEQ.ID.NO: 59) |
| huCx31.1 | LYVFHSFYPKYILPPVVKCHADPCPNIVDCFISKPSEKNIFTL | (SEQ.ID.NO: 60) |
| huCx32 | MYVFYLLYPGYAMVRLVKCDVYPCPNTVDCFVSRPTEKTVFTV | (SEQ.ID.NO: 61) |
| huCx36 | LYGWTMEPVFVCQRAPCPYLVDCFVSRPTEKTIFII | (SEQ.ID.NO: 62) |
| huCx37 | LYGWTMEPVFVCQRAPCPYLVDCFVSRPTEKTIFII | (SEQ.ID.NO: 63) |
| huCx40.1 | GALHYFLFGFLAPKKFPCTRPPCTGVVDCYVSRPTEKSLLML | (SEQ.ID.NO: 64) |
| huCx46 | IAGQYFLYGFELKPLYRCDRWPCPNTVDCFISRPTEKTIFII | (SEQ.ID.NO: 65) |
| huCx46.6 | LVGQYLLYGFEVRPFFPCSRQPCPHVVDCFVSRPTEKTVFLL | (SEQ.ID.NO: 66) |
| huCx40 | IVGQYFIYGIFLTTLHVCRRSPCPHPVNCYSRPTEKNVFIV | (SEQ.ID.NO: 67) |
| huCx45 | LIGQYFLYGFQVHPFYVCSRLPCHPKIDCFISRPTEKTIFLL | (SEQ.ID.NO: 68) |

Table 5 provides peptides inhibitors of connexin 40 shown with reference to the extracellular loops (E1 and E2) of connexin 40. The bold amino acids are directed to the transmembrane regions of connexin 40.

**Table 5. Cx40 peptide inhibitors**

| **E1** | | | | | | | | |
|---|---|---|---|---|---|---|---|---|
| | **LGTAA**ESSWGDEQADFRCDTIQPGCQNVCTDQAFPISHIR**FWVLQ** | | | | | | | (SEQ.ID.NO: 69) |
| | LGTAAESSWGDEQA | | | | | | | (SEQ.ID.NO: 70) |
| | | | DEQADFRCDTIQP | | | | | (SEQ.ID.NO: 71) |
| | | | | | TIQPGCQNVCTDQ | | | (SEQ.ID.NO: 72) |
| | | | | | | VCTDQAFPISHIR | | (SEQ.ID.NO: 73) |
| | | | | | | | AFPISHIRFWVLQ | (SEQ.ID.NO: 74) |

| **E2** | | | | | | | | |
|---|---|---|---|---|---|---|---|---|
| | **MEVGF**IVGQYFIYGIFLTTLHVCRRSPCPHPVNCYVSRPTEKN**VFIV** | | | | | | | (SEQ.ID.NO: 75) |
| | MEVGFIVGQYF | | | | | | | (SEQ.ID.NO: 76) |
| | | IVGQYFIYGIFL | | | | | | (SEQ.ID.NO: 77) |
| | | | | GIFLTTLHVCRRSP | | | | (SEQ.ID.NO: 78) |
| | | | | | RRSPCPHPVNCY | | | (SEQ.ID.NO: 79) |
| | | | | | | **VNCYVSRPTEKN** | | (SEQ.ID.NO: 80) |
| | | | | | | | **SRPTEKNVFIV** | (SEQ.ID.NO: 81) |

Table 6 provides peptides inhibitors of connexin 45 shown with reference to the extracellular loops (E1 and E2) of connexin 45. The bold amino acids are directed to the transmembrane regions of connexin 45.

**Table 6. Cx45 peptide inhibitors**

| **E1** | | | | | | | | | | | |
|---|---|---|---|---|---|---|---|---|---|---|---|
| | **LTAVG**GESIYYDEQSKFVCNTEQPGCENVCYDAFAPLSHVR**FWVFQ** | | | | | | | | | | (SEQ.ID.NO: 82) |
| | **LTAVG**GESIYYDEQS | | | | | | | | | | (SEQ.ID.NO: 83) |
| | | | DEQSKFVCNTEQP | | | | | | | | (SEQ.ID.NO: 84) |
| | | | | | TEQPGCENVCYDA | | | | | | (SEQ.ID.NO: 85) |
| | | | | | | | VCYDAFAPLSHVR | | | | (SEQ.ID.NO: 86) |
| | | | | | | | | | APLSHVRFWVFQ | | (SEQ.ID.NO: 87) |

| **E2** | | | | | | | | | | | |
|---|---|---|---|---|---|---|---|---|---|---|---|
| | **FEVGF**LIGQYFLYGFQVHPFYVCSRLPCHPKIDCFISRPTEKT**IFLL,** | | | | | | | | | | (SEQ.ID.NO: 88) |
| | FEVGFLIGQYF | | | | | | | | | | (SEQ.ID.NO: 89) |
| | | LIGQYFLYGFQV | | | | | | | | | (SEQ.ID.NO: 90) |
| | | | | GFQVHPFYVCSRLP | | | | | | | (SEQ.ID.NO: 91) |
| | | | | | | SRLPCHPKIDCF | | | | | (SEQ.ID.NO: 92) |
| | | | | | | | | **IDCFISRPTEKT** | | | (SEQ.ID.NO: 93) |
| | | | | | | | | | | **SRPTEKTIFLL** | (SEQ.ID.NO: 94) |

In certain embodiments, it is preferred that certain peptide inhibitors block hemichannels without a desired blocking of gap junctions. While not wishing to be bound to any particular theory or mechanism, it is also believed that certain peptidomimetics (e.g. VCYDKSFPISHVR, (SEQ.ID.NO: 11) block hemichannels without causing uncoupling of gap junctions. The peptide SRPTEKTIFII (SEQ.ID.NO: 7) may also be used, for example to block hemichannels without uncoupling of gap junctions. The peptide SRGGEKNVFIV (SEQ.ID.NO: 95) may be used that as a control sequence (DeVriese et al., Kidney Internat. 61: 177-185 (2002)). Examples of peptide inhibitors for connexin 45 YVCSRLPCHP (SEQ.ID.NO:96), QVHPFYVCSRL (SEQ.ID.NO:97), FEVGFLIGQYFLY (SEQ.ID.NO:98), GQYFLYGFQVHP (SEQ.ID.NO:99), GFQVHPFYVCSR (SEQ.ID.NO:100), AVGGESIYYDEQ (SEQ.ID.NO:101), YDEQSKFVCNTE (SEQ.ID.NO: 102), NTEQPGCENVCY (SEQ.ID.NO: 103), CYDAFAPLSHVR (SEQ.ID.N0:104), FAPLSHVRFWVF (SEQ.ID.NO:105) and LIGQY (SEQ.ID.N0:106), QVHPF (SEQ.ID.NO:107), YVCSR (SEQ.ID.N0:108), SRLPC (SEQ.ID.N0:109), LPCHP (SEQ.ID.NO:110) and GESIY (SEQ.ID.NO:111), YDEQSK (SEQ.ID.NO:112), SKFVCN (SEQ.ID.N0:113), TEQPGCEN (SEQ.ID.N0:114), VCYDAFAP (SEQ.ID.NO:115), LSHVRFWVFQ (SEQ.ID.N0:116) The peptides may only be 3 amino acids in length, including SRL, PCH, LCP, CHP, IYY, SKF, QPC, VCY, APL, HVR, or longer, for example: LIQYFLYGFQVHPF (SEQ.ID.NO: 117), VHPFYCSRLPCHP (SEQ.ID.N0:118), VGGESIYYDEQSKFVCNTEQPG (SEQ.ID.N0:119), TEQPGCENVCYDAFAPLSHVRF (SEQ.ID.N0:120), AFAPLSHVRFWVFQ (SEQ.ID.NO: 121).

Exemplary peptides may also include those reported in W02003032964 to Griffith, including, for example, the synthetic peptides homologous to one or more of the Gap 26 and 27 extracellular loop portions of a connexin protein, VCYDQAFPISHIR (SEQ.ID.NO: 122), VCYDKSFPISHVR (SEQ.ID.NO: 123), SRPTEKTIFII (SEQ.ID.NO: 124), SRPTEKNVFIV (SEQ.ID.NO: 125), RVDCFLSRPTEK (SEQ.ID.NO: 126), PVNCYVSRPTEK (SEQ.ID.NO: 127), IVDCYVSRPTEK (SEQ.ID.NO: 128), SRPTEKT (SEQ.ID.NO: 129), VCYDQAFPISHIR (SEQ.ID.NO: 130), VCYDKSFPISHVRI (SEQ.ID.NO: 131), SRPTEKTIFII (SEQ.ID.NO: 132), SRPTEKNVFIV (SEQ.ID.NO: 133), RVDCFLSRPTEK (SEQ.ID.NO: 134), PVNCYVSRPTEK (SEQ.ID.NO: 135), and IVDCYVSRPTEK (SEQ.ID.NO: 136), SRPTEKT (SEQ.ID.NO: 137).

Homology and homologues are discussed herein. Such a polypeptide typically has at least about 70% homology, preferably at least about 80, at least about 90%, at least about 95%, at least about 97% or at least about 99% homology with the relevant sequence, for example over a region of at least about 15, at least about 20, at least about 40, at least about 100 more contiguous polypeptide.

Homology may be calculated based on any method in the art. For example the UWGCG Package provides the BESTFIT program which can be used to calculate homology (for example used on its default settings) (Devereux et al (1984) Nucleic Acids Research 12:387-395). The PILEUP and BLAST algorithms can be used to calculate homology or line up sequences (typically on their default settings), for example as described in Altschul S. F. (1993) J Mol Evol 36:290-300*;* Altschul, S, F et al. (1990) J Mol Biol 215:403-10.

Software for performing BLAST analyses is publicly available through the National Center for Biotechnology Information (http://www.ncbi.nlm.nih.gov/). This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold (Altschul et al, supra). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached.

The BLAST algorithm parameters W, T and X determine the sensitivity and speed of the alignment. The BLAST program uses as defaults a word length (W) of11, the BLOSUM62 scoring matrix (see Henikoff and Henikoff (1992) Proc.Natl. Acad. Sci. USA 89: 10915-10919) alignments (B) of 50, expectation (E) of 10, M=5, N=4, and a comparison of both strands.

The BLAST algorithm performs a statistical analysis of the similarity between two sequences; see e.g., Karlin and Altschul (1993) Proc. Natl.Acad. Sci. USA 90: 5873-5787. One measure of similarity provided by the BLAST algorithm is the smallest sum probability (P(N)), which provides an indication of the probability by which a match between two amino acid sequences would occur by chance. For example, a sequence is considered similar to another sequence if the smallest sum probability in comparison of the first sequence to a second sequence is less than about 1, preferably less than about 0.1, more preferably less than about 0.01, and most preferably less than about 0.001.

The homologous sequence typically differs from the relevant sequence by at least about (or by no more than) 2, 5, 10, 15, 20 more mutations (which may be substitutions, deletions or insertions). These mutations may be measured across any of the regions mentioned above in relation to calculating homology.

The homologous sequence typically hybridises selectively to the original sequence at a level significantly above background. Selective hybridization is typically achieved using conditions of medium to high stringency). However, such hybridisation may be carried out under any suitable conditions known in the art (see Sambrook et al (1989), Molecular Cloning: A Laboratory Manual).

### Gap Junction Modifying Agents - Other Anti-connexin Agents

Gap junction modulation agents, include agents that close or block gap junctions and/or hemichannels or otherwise prevent or decrease cell to cell communication via gap junctions or prevent or decrease cell communication to the extracellular environment via hemichannels. They include agents or compounds that prevent, decrease or inhibit, in whole or in part, the activity, function, or formation of a hemichannel or a gap junction.

In certain embodiments, a gap junction modulation agent induces closure, in whole or in part, of a hemichannel or a gap junction. In other embodiments, a gap junction modifying agent blocks, in whole or in part, a hemichannel or a gap junction. In certain embodiments, a gap junction modifying agent decreases or prevents, in whole or in part, the opening of a hemichannel or gap junction.

In certain embodiments, said blocking or closure of a gap junction or hemichannel by a gap junction modifying agent can reduce or inhibit extracellular hemichannel communication by preventing or decreasing the flow of small molecules through an open channel to and from an extracellular or periplasmic space.

Gap junction modifying agents used for closing hemichannels or gap junctions (e.g. phosphorylating connexin 43 tyrosine residues) have been reported in U.S. Pat. No. 7,153,822 to Jensen et al., U.S. Pat. No. 7,250,397, and assorted patent publications. See also Gourdie et al., see W02006069181, with regard to connexin carboxy-terminal polypeptides that are said to, for example, inhibit ZO-1 protein binding. Gourdie et al, W02006069181 1 describes use of formulations comprising such peptides.

As used herein, "gap junction phosphorylating agent" may include those agents or compounds capable of inducing phosphorylation on connexin amino acid residues in order to induce gap junction or hemichannel closure. Exemplary sites of phosphorylation include one or more of a tyrosine, serine or threonine residues on the connexin protein. In certain embodiments, modulation of phosphorylation may occur on one or more residues on one or more connexin proteins. Exemplary gap junction phosphorylating agents are well known in the art and may include, for example, c-Src tyrosine kinase or other G protein-coupled receptor agonists. See Giepmans B, J. Biol. Chem., Vol. 276, Issue 11, 8544-8549, March 16, 2001. In one embodiment, modulation of phosphorylation on one or more of these residues impacts hemichannel function, particularly by closing the hemichannel. In another embodiment, modulation of phosphorylation on one or more of these residues impacts gap junction function, particularly by closing the gap junction. Gap junction phosphorylating agents that target the closure of connexin 43 gap junctions and hemichannels are preferred.

Still other anti-connexin agents include connexin carboxy-terminal polypeptides. See Gourdie et al., WO2006/069181.

In certain another aspect, gap junction modifying agent may include, for example, aliphatic alcohols; octanol; heptanol; anesthetics (e.g. halothane), ethrane, fluothane, propofol and thiopental; anandamide; arylaminobenzoate (FFA: flufenamic acid and similar derivatives that are lipophilic); carbenoxolone; Chalcone: (2',5'-dihydroxychalcone); CHFs (Chlorohydroxyfuranones); CMCF (3-chloro-4-(chloromethyl)-5-hydroxy-2(5H)-furanone); dexamethasone; doxorubicin (and other anthraquinone derivatives); eicosanoid thromboxane A(2) (TXA(2)) mimetics; NO (nitric oxide); Fatty acids (e.g. arachidonic acid, oleic acid and lipoxygenase metabolites; Fenamates (flufenamic (FFA), niflumic (NFA) and meclofenamic acids (MFA)); Genistein; glycyrrhetinic acid (GA):18a-glycyrrhetinic acid and 18-beta - glycyrrhetinic acid, and derivatives thereof; lindane; lysophosphatidic acid; mefloquine; menadione; 2-Methyl-1,4-naphthoquinone, vitamin K(3); nafenopin; okadaic acid; oleamide; oleic acid; PH, gating by intracellular acidification; e.g. acidifying agents; polyunsaturated fatty acids; fatty acid GJIC inhibitors (e.g. oleic and arachidonic acids); quinidine; quinine; all trans-retinoic acid; and tamoxifen.

### Dosage, Formulation and Administration

The gap junction modulating agents of the invention (typically in the form of the formulations discussed herein) may be administered to a subject in need of treatment, such as a subject with any of the wounds mentioned herein. The wound of the subject can thus be improved. The gap junction modulating agents and formulations may thus be used in the treatment of the subject's body by therapy. They may be used in the manufacture of a medicament to treat any of the wounds mentioned herein.

Thus, in accordance with the invention, there are provided formulations by which cell-cell and/or hemichannel communication can be downregulated in a transient and site-specific manner. The gap junction modulating agent may be conveniently formulated with a pharmaceutically acceptable carrier to give the desired final concentration.

The gap junction modulating agent may be present in a substantially isolated form. It will be understood that the product may be mixed with carriers or diluents which will not interfere with the intended purpose of the product and still be regarded as substantially isolated. A product of the invention may also be in a substantially purified form, in which case it will generally comprise about 80%, 85% or 90%, e.g. at least about 95%, at least about 98% or at least about 99% of the peptide (or other gap junction modulating agent) or dry mass of the preparation.

Depending on the intended route of administration, the pharmaceutical products, pharmaceutical compositions, combined preparations and medicaments of the invention may, for example, take the form of solutions, suspensions, instillations, salves, creams, gels, foams, sprays, ointments, emulsions, lotions, paints, sustained release formulations, or powders, and typically contain .01% to about 1% of active ingredient(s), about 1 %-50% or active ingredient(s), about 2%-60% of active ingredient(s), about 2%-70% of active ingredient(s), or up to about 90% of active ingredient(s). Other suitable formulations include pluronic gel-based formulations, carboxymethylcellulose(CMC)-based formulations, and hyroxypropylmethylcellulose(HPMC)-based formulations. Suitable formulations including pluronic gel, for example comprise from about 10 to about 15 percent, suitably about 12 percent. Other useful formulations include slow or delayed release preparations.

Gels or jellies may be produced using a suitable gelling agent including, but not limited to, gelatin, tragacanth, or a cellulose derivative and may include glycerol as a humectant, emollient, and preservative. Ointments are semi-solid preparations that consist of the active ingredient incorporated into a fatty, waxy, or synthetic base. Examples of suitable creams include, but are not limited to, water-in-oil and oil-in-water emulsions. Water-in-oil creams may be formulated by using a suitable emulsifying agent with properties similar, but not limited, to those of the fatty alcohols such as cetyl alcohol or cetostearyl alcohol and to emulsifying wax. Oil-in-water creams may be formulated using an emulsifying agent such as cetomacrogol emulsifying wax. Suitable properties include the ability to modify the viscosity of the emulsion and both physical and chemical stability over a wide range of pH. The water soluble or miscible cream base may contain a preservative system and may also be buffered to maintain an acceptable physiological pH.

Foam preparations may be formulated to be delivered from a pressurized aerosol canister, via a suitable applicator, using inert propellants. Suitable excipients for the formulation of the foam base include, but are not limited to, propylene glycol, emulsifying wax, cetyl alcohol, and glyceryl stearate. Potential preservatives include methylparaben and propylparaben.

Preferably the gap junction modulating agents are combined with a pharmaceutically acceptable carrier or diluent to produce a pharmaceutical composition. Suitable carriers and diluents include isotonic saline solutions, for example phosphate-buffered saline. Suitable diluents and excipients also include, for example, water, saline, dextrose, glycerol, or the like, and combinations thereof. In addition, if desired substances such as wetting or emulsifying agents, stabilizing or ph buffering agents may also be present.

The term "pharmaceutically acceptable carrier" refers to any pharmaceutical carrier that does not itself induce the production of antibodies harmful to the individual receiving the composition, and which can be administered without undue toxicity. Suitable carriers can be large, slowly metabolized macromolecules such as proteins, polysaccharides, polylactic acids, polyglycolic acids, polymeric amino acids, and amino acid copolymers.

Pharmaceutically acceptable salts can also be present, e.g., mineral acid salts such as hydrochlorides, hydrobromides, phosphates, sulfates, and the like; and the salts of organic acids such as acetates, propionates, malonates, benzoates, and the like.

Suitable carrier materials include any carrier or vehicle commonly used as a base for creams, lotions, sprays, foams, gels, emulsions, lotions or paints for topical administration. Examples include emulsifying agents, inert carriers including hydrocarbon bases, emulsifying bases, non-toxic solvents or water-soluble bases. Particularly suitable examples include pluronics, HPMC, CMC and other cellulose-based ingredients, lanolin, hard paraffin, liquid paraffin, soft yellow paraffin or soft white paraffin, white beeswax, yellow beeswax, cetostearyl alcohol, cetyl alcohol, dimethicones, emulsifying waxes, isopropyl myristate, microcrystalline wax, oleyl alcohol and stearyl alcohol.

Preferably, the pharmaceutically acceptable carrier or vehicle is a gel, suitably a nonionic polyoxyethylene-polyoxypropylene copolymer gel, for example, a Pluronic gel, preferably Pluronic F-127 (BASF Corp.). This gel is particularly preferred as it is a liquid at low temperatures but rapidly sets at physiological temperatures, which confines the release of the ODN component to the site of application or immediately adjacent that site.

An auxiliary agent such as casein, gelatin, albumin, glue, sodium alginate, carboxymethylcellulose, methylcellulose, hydroxyethylcellulose or polyvinyl alcohol may also be included in the formulation of the invention.

Other suitable formulations include pluronic gel-based formulations, carboxymethylcellulose(CMC)-based formulations, and hyroxypropylmethylcellulose(HPMC)-based formulations. The composition may be formulated for any desired form of delivery, including topical, instillation, parenteral, intramuscular, subcutaneous, or transdermal administration. Other useful formulations include slow or delayed release preparations.

The effective dose for a given subject or wound can be determined by routine experimentation or other methods known in the art or later developed. For example, in order to formulate a range of dosage values, cell culture assays and animal studies can be used. The dosage of such compounds preferably lies within the dose that is therapeutically effective for at least 50% of the population, and that exhibits little or no toxicity at this level.

The effective dosage of each of the gap junction modulating agents employed in the methods and compositions of the invention may vary depending on a number of factors including the particular gap junction modulating agent or agents employed, the mode of administration, the frequency of administration, the wound being treated, the severity of the wound being treated, the route of administration, the needs of a patient sub-population to be treated or the needs of the individual patient which different needs can be due to age, sex, body weight, relevant medical wound specific to the patient.

The dose at which an gap junction modulating agent is administered to a patient will depend upon a variety of factors such as the age, weight and general wound of the patient, the wound that is being treated, and the particular gap junction modulating agent that is being administered.

A suitable dose may be based on the amount of gap junction modulating agent per kg of body weight of the subject, and include, from about 0.001 to about 100 mg/kg body weight such as about 0.01 to about 40 mg/kg body weight. A suitable dose may however be from about 0.001 to about 0.1 mg/kg body weight such as about 0.01 to about 0.050 mg/kg body weight.

A suitable dose may be from about 0.001 to about 0.1 mg/kg body weight such as about 0.01 to about 0.1 mg/kg body weight. A suitable dose may however be from about 0.001 to about 0.25 mg/kg body weight, including about 0.001 to about 0.050 mg/kg body weight. Alternatively, for example, in certain embodiments, the dosage of each of the subject compounds will generally be in the range of about 1 ng to about 1 microgram per kg body weight, about 1 ng to about 0.1 microgram per kg body weight, about 1 ng to about 10 ng per kg body weight, about 10 ng to about 0.1 microgram per kg body weight, about 0.1 microgram to about 1 microgram per kg body weight, about 20 ng to about 100 ng per kg body weight, about 0.001 mg to about 100 mg per kg body weight, about 0.01 mg to about 10 mg per kg body weight, or about 0.1 mg to about 1 mg per kg body weight. In certain embodiments, the dosage of each of the subject compounds will generally be in the range of about 0.001 mg to about 0.01 mg per kg body weight, about 0.01 mg to about 0.1 mg per kg body weight, about 0.1 mg to about 1 mg per kg body weight, or about 1 mg to about 10 mg per kg body weight. If more than one gap junction modulating agent is used, the dosage of each gap junction modulating agent need not be in the same range as the other.

Doses from about 1 to 100, 200, 300, 400, 500 micrograms, and up to 1 milligram per application are appropriate. Gap junction modulating agent dose amounts include, for example, about 1, about 2, about 3, about 4, or about 5 micrograms, from about 5 to about 10 micrograms, from about 10 to about 15 micrograms, from about 15 to about 20 micrograms, from about 20 to about 30 micrograms, from about 30 to about 40 micrograms, from about 40 to about 50 micrograms, from about 50 to about 75 micrograms, from about 75 to about 100 micrograms, from about 100 micrograms to about 250 micrograms, and from 250 micrograms to about 500 micrograms. Dose amounts from about 0.5 to about 1.0 milligrams or more or also provided.

Alternatively, the dosage of each of the gap junction modulating agents in the compositions of the subject invention may be determined by reference to the composition's concentration relative to the size, length, depth, area or volume of the area to which it will be applied. For example, in certain topical applications, dosing of the pharmaceutical compositions may be calculated based on mass (e.g. grams) of or the concentration in a pharmaceutical composition (*e.g.* µg/ul) per length, depth, area, or volume of the area of application. Thus, other useful doses range from about 1 to about 10 micrograms per square centimeter of wound size. Certain doses will be about 1-2, about 1-5, about 2-4, about 5-7, and about 8-10 micrograms per square centimeter of wound size. Other useful doses are greater than about 10 micrograms per square centimeter of wound size, including about 15 micrograms per square centimeter of wound size, about 20 micrograms per square centimeter of wound size, about 25 micrograms per square centimeter of wound size, about 30 micrograms per square centimeter of wound size, about 35 micrograms per square centimeter of wound size, about 40 micrograms per square centimeter of wound size, about 50 micrograms per square centimeter of wound size, and about 100 about 150 micrograms per square centimeter of wound size. Other doses include about 150-200 micrograms per square centimeter, about 200-250 micrograms per square centimeter, about 250-300 micrograms per square centimeter, about 300-350 micrograms per square centimeter, about 350-400 micrograms per square centimeter, and about 400-500 micrograms per square centimeter. Still other doses include doses ranging form 500-1000 micrograms per square centimeter. Other doses may be 1-10 milligrams per square centimeter. Sustained release formulations may be particularly appropriate for anti-connexin peptides and peptidomimetics.

In certain embodiments, the gap junction modulating agent composition may be applied at about 0.01 micromolar (µM) or 0.05 µM to about 200 µM, or up to 300 µM or up to 1000 µM, or up to 2000 µM or up to 3200 µM or more, final concentration at the treatment site and/or adjacent to the treatment site, and any doses and dose ranges within these dose numbers. Preferably, the gap junction modulating agents, including anti-connexin peptides or peptidomimetics composition is applied at about 0.05 µM to about 100 µM final concentration, more preferably, the gap junction modulating agent composition is applied at about 1.0 µM to about 50 µM final concentration, and more preferably, the gap junction modulating agent composition is applied at about 5-10 µM to about 30-50 µM final concentration. Additionally, the combined gap junction modulating agent composition is applied at about 8 µM to about 20 µM final concentration, and alternatively the gap junction modulating agent composition is applied at about 10 µM to about 20 µM final concentration, or at about 10 to about 15 µM final concentration. In certain other embodiments, the gap junction modulating agent is applied at about 10 µM final concentration. In other embodiments, the anti-connexin agent is applied at about a 20 µM, 30 µM, 40 µM, 50 µM, 60 µM, 70 µM, 80 µM, 90 µM, 100 µM., 10-200 µM, 200-300 µM, 300-400 µM, 400-500 µM, 500-600 µM, 600-700 µM, 700-800 µM, 800-900 µM, or 900-1000 µM or 1000 - 1500 µM, or 1500 - 2000 µM or 2000 - 3000 µM or greater final concentration, or any dose within these ranges.

In yet another embodiment, the gap junction modulating agent composition is applied at about 1-15 µM final concentration.

Gap junction modulating agent dose amounts include, for example, about 0.1-1, 1-2, 2-3, 3-4, or 4-5 micrograms (µg), from about 5 to about 10 µg, from about 10 to about 15 µg, from about 15 to about 20 µg, from about 20 to about 30 µg, from about 30 to about 40 µg, from about 40 to about 50 µg, from about 50 to about 75 µg, from about 75 to about 100 µg, from about 100 µg to about 250 µg, and from 250 µg to about 500 µg. Dose amounts from 0.5 to about 1.0 milligrams or more or also provided, as noted above.

Dose volumes will depend on the size of the site to be treated, and may range, for example, from about 25-100 µL to about 100-200 µL, from about 200-500 µL to about 500-1000 µL. Milliliter doses are also appropriate for larger treatment sites.

Conveniently, the composition is administered in a sufficient amount to downregulate expression of said connexin proteins, or modulate gap junction formation or connexon opening for at least about 0.5 to 1 hour, at least about 1-2 hours, at least about 2-4 hours, at least about 4-6 hours, at least about 6-8 hours, at least about 8-10 hours, at least about 12 hours, or at least about 24 hours post-administration.

The dosage of each of the gap junction modulating agents in the compositions and methods of the subject invention may also be determined by reference to the concentration of the composition relative to the size, length, depth, area or volume of the area to which it will be applied. For example, in certain topical and other applications, e.g., instillation, dosing of the pharmaceutical compositions may be calculated based on mass (e.g. micrograms) of or the concentration in a pharmaceutical composition (e.g. µg/µl) per length, depth, area, or volume of the area of application.

The combined use of several gap junction modulating agents may reduce the required dosage for any individual component because the onset and duration of effect of the different components may be complementary. In such combined therapy, the different active agents may be delivered together or separately, and simultaneously or at different times within the day.

The doses may be administered in single or divided applications. The doses may be administered once, or application may be repeated. Repeat applications are typically applied about once per week, or when wound-healing may appear to be stalled or slowing. Doses may be applied from 1 to 7 days apart, or longer. Repeat applications may be made, for example, weekly, or bi-weekly, or monthly or in other frequency for example if and when wound healing slows or is stalled. Alternatively, for some indications, such as certain ocular uses, more frequent dosing, up to hourly may be employed.

One or more gap junction modulating agents may be administered by the same or different routes. Preferably one or more gap junction modulating agents are delivered by topical administration (peripherally or directly to a site), including but not limited to topical administration using solid supports (such as dressings and other matrices) and medicinal formulations (such as gels, mixtures, suspensions and ointments). In one embodiment, the solid support comprises a biocompatible membrane or insertion into a treatment site. In another embodiment, the solid support comprises a dressing or matrix. In one embodiment of the invention, the solid support composition may be a slow release solid support composition, in which the one or more gap junction modulating agents is dispersed in a slow release solid matrix such as a matrix of alginate, collagen, or a synthetic bioabsorbable polymer. Preferably, the solid support composition is sterile or low bio-burden. In one embodiment, a wash solution comprising one or more gap junction modulating agents can be used.

The delivery of the gap junction modulating agent component of the formulation may occur over a period of time, in some instances for about 1-2 hours, about 2-4 hours, about 4-6 hours, about 6-8, or about 24 hours or longer, may be a particular advantage in certain orthopedic treatments or more severe injuries or wounds or in treatment of chronic or delayed healing wounds. In some instances, cell loss may extend well beyond the site of a procedure to surrounding cells. Such loss may occur within 24 hours of the original procedure and is mediated by gap junction cell-cell communication. Administration of gap junction modulating agent(s), e.g., for downregulation of connexin expression, or blockade of connexon opening, therefore will modulate communication between the cells, or loss into the extracellular space in the case of connexon regulation, and minimize additional cell loss or injury or consequences of injury.

While the delivery period will be dependent upon both the site at which the downregulation is to be induced and the therapeutic effect which is desired, continuous or slow-release delivery for about 1-2 hours, about 2-4 hours, about 4-6 hours, about 6-8, or about 24 hours or longer is provided. In accordance with the present invention, this is achieved by inclusion of the gap junction modulating agents in a formulation together with a pharmaceutically acceptable carrier or vehicle.

According to an aspect, the gap junction modulating agents may be administered topically (at the site to be treated). In one aspect, the gap junction modulating agents are combined with a pharmaceutically acceptable carrier or diluent to produce a pharmaceutical composition. Suitable carriers and diluents include isotonic saline solutions, for example phosphate-buffered saline. In another aspect, the composition may be formulated for intramuscular, subcutaneous, or transdermal administration.

The routes of administration and dosages described herein are intended only as a guide for a dosage for any particular patient and wound.

### Dressings and Matrices

In one aspect, the one or more gap junction modulating agents are provided in the form of a dressing or matrix. In certain embodiments, the one or more gap junction modulating agents are provided in the form of a liquid, semi solid or solid composition for application directly, or the composition is applied to the surface of, or incorporated into, a solid contacting layer such as a dressing gauze or matrix. The dressing composition may be provided for example, in the form of a fluid or a gel. The one or more gap junction modulating agents may be provided in combination with conventional pharmaceutical excipients for topical application. Suitable carriers include: Pluronic gels, Polaxamer gels, Hydrogels containing cellulose derivatives, including hydroxyethyl cellulose, hydroxymethyl cellulose, carboxymethyl cellulose, hydroxypropylmethyl cellulose and mixtures thereof; and hydrogels containing polyacrylic acid (Carbopols). Suitable carriers also include creams/ointments used for topical pharmaceutical preparations, e.g., creams based on cetomacrogol emulsifying ointment. The above carriers may include alginate (as a thickener or stimulant), preservatives such as benzyl alcohol, buffers to control pH such as disodium hydrogen phosphate/sodium dihydrogen phosphate, agents to adjust osmolarity such as sodium chloride, and stabilizers such as EDTA.

In one embodiment one or more anti-connexin peptides or peptidomimetics, for example an anti-connexin 43 peptides or peptidomimetics, is administered on a natural or synthetic matrix.

Suitable dressings or matrices may include, for example, the following with one or more gap junction modulating agents. Connexin 43 gap junction modulating agents are preferred:

1) Absorptives: suitable absorptives may include, for example, absorptive dressings, which can provide, for example, a semi-adherent quality or a non-adherent layer, combined with highly absorptive layers of fibers, such as for example, cellulose, cotton or rayon. Alternatively, absorptives may be used as a primary or secondary dressing.

2) Alginates: suitable alginates include, for example, dressings that are non-woven, non-adhesive pads and ribbons composed of natural polysaccharide fibers or xerogel derived from seaweed. Suitable alginates dressings may, for example, form a moist gel through a process of ion exchange upon contact with exudate. In certain embodiments, alginate dressings are designed to be soft and conformable, easy to pack, tuck or apply over irregular-shaped areas. In certain embodiments, alginate dressings may be used with a second dressing.

3) Antimicrobial Dressings: suitable antimicrobial dressings may include, for example, dressings that can facilitate delivery of bioactive agents, such as, for example, silver and polyhexamethylene biguanide (PHMB), to maintain efficacy against infection, where this is needed or desireable. In certain embodiments, suitable antimicrobial dressings may be available as for example, as sponges, impregnated woven gauzes, film dressings, absorptive products, island dressings, nylon fabric, non-adherent barriers, or a combination of materials.

4) Biological & Biosynthetics: suitable biological dressings or biosynthetic dressings may include, for example, gels, solutions or semi-permeable sheets derived from a natural source. In certain embodiments, a gel or solution is applied to the treatment site and covered with a dressing for barrier protection. In another embodiment, a sheet is placed in situ which may act as membrane, remaining in place after a single application.

5) Collagens: suitable collagen dressings may include, for example, gels, pads, particles, pastes, powders, sheets or solutions derived from for example, bovine, porcine or avian sources or other natural sources or donors. In certain embodiments, the collagen dressing may interact with treatment site exudate to form a gel. In certain embodiments, collagen dressing may be used in combination with a secondary dressing.

6) Composites: suitable composite dressings may include, for example, dressings that combine physically distinct components into a single product to provide multiple functions, such as, for example, a bacterial barrier, absorption and adhesion. In certain embodiment, the composite dressings are comprised of, for example, multiple layers and incorporate a semi-or non-adherent pad. In certain embodiment, the composite may also include for example, an adhesive border of non-woven fabric tape or transparent film. In certain other embodiment, the composite dressing may function as for example, either a primary or a secondary dressing and in yet another embodiments, the dressing may be used in combination with topical pharmaceutical composition.

7) Contact Layers: suitable contact layer dressings may include, for example, thin, non-adherent sheets placed on an area to protect tissue from for example, direct contact with other agents or dressings applied to the treatment site. In certain embodiments, contact layers may be deployed to conform to the shape of the area of the treatment site and are porous to allow exudate to pass through for absorption by an overlying, secondary dressing. In yet another embodiment, the contact layer dressing may be used in combination with topical pharmaceutical composition.

8) Elastic Bandages: suitable elastic bandages may include, for example, dressings that stretch and conform to the body contours. In certain embodiment, the fabric composition may include for example, cotton, polyester, rayon or nylon. In certain other embodiments, the elastic bandage may for example, provide absorption as a second layer or dressing, to hold a cover in place, to apply pressure or to cushion a treatment site.

9) Foams: suitable foam dressings may include, for example, sheets and other shapes of foamed polymer solutions (including polyurethane) with small, open cells capable of holding fluids. Exemplary foams may be for example, impregnated or layered in combination with other materials. In certain embodiment, the absorption capability may be adjusted based on the thickness and composition of the foam. In certain other embodiments, the area in contact with the treatment site may be non-adhesive for easy removal. In yet another embodiment, the foam may be used in combination with an adhesive border and/or a transparent film coating that can serve as an anti-infective barrier.

10) Gauzes & Non-Woven dressings: suitable gauze dressings and woven dressings may include, for example, dry woven or non-woven sponges and wraps with varying degrees of absorbency. Exemplary fabric composition may include, for example, cotton, polyester or rayon. In certain embodiment, gauzes and non-woven dressing may be available sterile or non-sterile in bulk and with or without an adhesive border. Exemplary gauze dressings and woven dressings may be used for cleansing, packing and covering a variety of treatment sitez.

11) Hydrocolloids: suitable hydrocolloid dressings may include, for example, wafers, powders or pastes composed of gelatin, pectin or carboxymethylcellulose. In certain embodiment, wafers are self-adhering and available with or without an adhesive border and in a wide variety of shapes and sizes. Exemplary hydrocolloids are useful on areas that require contouring. In certain embodiments, powders and pastes hydrocolloids may use used in combination with a secondary dressing.

12) Hydrogels (Amorphous): suitable amorphous hydrogel dressings may include, for example, formulations of water, polymers and other ingredients with no shape, designed to donate moisture and to maintain a moist healing environments and or to rehydrate the treatment site. In certain embodiment, hydrogels may be used in combination with a secondary dressing cover.

13) Hydrogels: Impregnated Dressings: suitable impregnated hydrogel dressings may include, for example, gauzes and non-woven sponges, ropes and strips saturated with an amorphous hydrogel. Amorphous hydrogels may include for example, formulations of water, polymers and other ingredients with no shape, designed to donate moisture to a dry treatment site and to maintain a moist healing environment.

14) Hydrogel Sheets: suitable hydrogel sheets may include for example, three-dimensional networks of cross-linked hydrophilic polymers that are insoluble in water and interact with aqueous solutions by swelling. Exemplary hydrogels are highly conformable and permeable and can absorb varying amounts of drainage, depending on their composition. In certain embodiment, the hydrogel is non-adhesive against the treatment site or treated for easy removal.

15) Impregnated Dressings: suitable impregnated dressings may include, for example, gauzes and non-woven sponges, ropes and strips saturated with a solution, an emulsion, oil, gel or some other pharmaceutically active compound or carrier agent, including for example, saline, oil, zinc salts, petrolatum, xeroform and scarlet red as well as the compounds described herein.

16) Silicone Gel Sheets: suitable silicone gel sheet dressings may include, for example, soft covers composed of cross-linked polymers reinforced with or bonded to mesh or fabric.

17) Solutions: suitable liquid dressings may include, for example, mixtures of multiprotein material and other elements found in the extracellular matrix. In certain embodiment, exemplary solutions may be applied to the treatment site after debridement and cleansing and then covered with an absorbent dressing or a nonadherent pad.

18) Transparent Films: suitable transparent film dressings may include polymer membranes of varying thickness coated on one side with an adhesive. In certain embodiments, transparent films are impermeable to liquid, water and bacteria but permeable to moisture vapor and atmospheric gases. In certain embodiments, the transparency allows visualization of the treatment site.

19) Fillers: suitable filler dressings may include, for example, beads, creams, foams, gels, ointments, pads, pastes, pillows, powders, strands or other formulations. In certain embodiment, fillers are non-adherent and may include a time-released antimicrobial. Exemplary fillers may be useful to maintain a moist environment, manage exudate, and for treatment of for example, partial- and full- thickness wounds, infected wounds, draining wounds and deep wounds that require packing.

Any of the methods of treating a subject having or suspected of having a disease, disorder, and/or condition, referenced or described herein may utilize the administration of any of the doses, dosage forms, formulations, and/or compositions herein described.

### Peptide and Other Gap Junction Modulating Compositions

The present invention relates to pharmaceutical compositions comprising gap junction modulating compounds (including peptidomimetics). The compositions are useful in enhancing or promoting healing of, for example, chronic wounds and delayed or difficult to heal wounds, including other wounds as described herein. Equally, in instances of other tissue damage the methods and compositions of the invention are effective in both promoting the wound healing process and reducing inflammation. Therefore, the formulations and compositions of the invention have clear benefit in the treatment of chronic and slow healing wounds, and other wounds as described herein.

In certain embodiments, the composition of the invention comprises an effective amount one or more gap junction modulating agents. In one embodiment, the composition contains a gap junction modulating agent targeted to a gap junction associated with one connexin only. Most preferably, this gap junction is associated with connexin 43 protein.

Alternatively, the compositions may comprise one or more gap junction modulating agents targeted to gap junctions associated with more than one connexin protein. Preferably, one of the connexin proteins to which modulators are directed is connexin 43. Other gap junction to which modulators are directed may include, for example, gap junctions associated with connexins 26, 30, 30.3, 31.1, 32, 36, 37, 40, 40.1, 44.6, 45 and 46.

In other embodiments, the composition of the invention comprises (a) an effective amount of one or more one or more gap junction modulating agents and (b) a pharmaceutically acceptable carrier or diluent. In one aspect the invention provides a composition comprising (a) one or more polypeptides and (b) a pharmaceutically acceptable carrier or diluent. In a preferred embodiment, the polypeptide is a connexin 26, 31.1, 32, 37, 40, 43 or 45 peptidomimetic or homolog thereof. In other embodiments, the polypeptide is a connexin 30, 30.3, 36, 40.1, 44.6, 45 or 46 peptidomimetic or homolog thereof.

In one aspect the invention provides a pharmaceutical composition comprising (a) one or more binding proteins and (b) a pharmaceutically acceptable carrier or diluent. In one embodiment the binding protein is an antibody. In a preferred embodiment, the binding protein is a monoclonal antibody, polyclonal antibody, antibody fragment, single chain antibodies, single chain Fvs, or single chain binding molecule. In one embodiment the binding protein, for example an antibody, specifically binds to a connexin polypeptide, preferably a connexin 26, connexin 30, connexin 31.1, connexin 32, connexin 37, connexin 40 or connexin 45 polypeptide or any part thereof. In one preferred embodiment the binding protein, for example an antibody, specifically binds to a connexin 43 polypeptide or any part thereof. In one embodiment the binding protein specifically binds to a connexin polypeptide and has an affinity of greater than or equal to about 10⁴ M⁻¹, greater than or equal to about 10⁶ M⁻¹, greater than or equal to about 10⁷ M⁻¹, or greater than or equal to about 10⁸ M⁻¹. Affinities of even greater than about 10⁸ M⁻¹ are suitable, such as affinities equal to or greater than about 10⁹ M⁻¹, about 10¹⁰ M⁻¹, about 10¹¹ M⁻¹, and about 10¹² M⁻¹.

In another embodiment, the pharmaceutical composition comprises (a) one or more binding proteins and (b) a pharmaceutically acceptable carrier or diluent, wherein the binding protein is a peptide inhibitor. In one embodiment, the peptide inhibitor comprises an amino acid sequence corresponding to a transmembrane region of connexin 26, connexin 30, connexin 31.1, connexin 32, connexin 37, connexin 40 or connexin 45. In a preferred embodiment, the peptide inhibitor comprises an amino acid sequence corresponding to a transmembrane region of connexin 43. In another embodiment, the peptide inhibitor comprises from about 5-20 contiguous amino acids set forth in SEQ.ID.NO:1. In another embodiment, the peptide inhibitor comprises from about 5-20 contiguous amino acids set forth in SEQ.ID.NO:2. In another embodiment, the peptide inhibitor comprises an amino acid sequence corresponding to the regions at positions 37-76 and 178-208 of SEQ.ID.NO:2. In another embodiment, the peptide inhibitor comprises from about 5-20 contiguous amino acids set forth in SEQ.ID.NO:3, SEQ.ID.NO:4, SEQ.ID.NO:5, SEQ.ID.NO:6, SEQ.ID.NO:7, SEQ.ID.NO:8, SEQ.ID.NO:9, SEQ.ID.NO:10, SEQ.ID.NO:11, SEQ.ID.NO:12, SEQ.ID.NO:13, SEQ.ID.NO:14, SEQ.ID.NO:15, SEQ.ID.NO:16, SEQ.ID.NO:17, SEQ.ID.NO:18, SEQ.ID.NO:19, SEQ.ID.NO:20, SEQ.ID.NO:21, SEQ.ID.NO:22, SEQ.ID.NO:23, SEQ.ID.NO:24, SEQ.ID.NO:25, or SEQ.ID.NO:26. In another embodiment, the peptide inhibitor comprises from about 8 to about 11 contiguous amino acids set forth in SEQ.ID.NOs: 27-54. In another embodiment, the peptide inhibitor comprises from about 8 to about 15 contiguous amino acids set forth in SEQ.ID.NOs: 18, 19, or 55-68. In yet another embodiment, the peptide inhibitor has one or more conservative amino acid changes.

### Treatment

The invention includes methods for treating and/or preventing, in whole or in part, various diseases, disorders and wounds, including, for example, methods of treating a wound that does not heal at the expected rate. These include delayed or difficult to heal wounds (including delayed or incompletely healing wounds), and chronic wounds. Examples of wounds that do not heal at the expected rate include ulcers such as diabetic ulcers, vascultic ulcers, arterial ulcers, venous ulcers, venous stasis ulcers, burn ulcers, infectious ulcers, trauma-induced ulcers, pressure ulcers and decubitus ulcers. Other wounds that do not heal at expected rates include dehiscent wounds.

The invention also include methods for treating and/or preventing, in whole or in part, chronic wounds characterized, in whole or in party, by (a) a chronic self-perpetuating state of wound inflammation, (b) a deficient and defective wound extracellular matrix, (c) poorly responding (senescent) wound cells (*e.g.* fibroblasts), limited extracellular matrix production, (d) a failure of re-epithelialization due in part to lack of the necessary extracellular matrix orchestration and lack of scaffold for migration, (e) prolonged inflammation and proteolytic activity, leading to ulcerative lesions, including for example, diabetic, pressure (decubitous), venous, and arterial ulcers, (f) progressive deposition of matrix in the affected area, (g) longer repair times, (h) less wound contraction, (i) slower re-epithelialization and/or (j) increased thickness of granulation tissue, comprising administering one or more gap junction modulating agents and a pharmaceutically acceptable carrier or diluent.

The invention also include methods for treating and/or preventing, in whole or in part, delayed or difficult to heal wounds, characterized, in whole or in part, by (a) a prolonged inflammatory phase, (b) a slow forming extracellular matrix, and/or (c) a decreased rate of epithelialization, comprising administering one or more gap junction modulating agents and a pharmaceutically acceptable carrier or diluent.

The invention also includes methods of treating and/or preventing, in whole or in part, arterial ulcers or ulcerations comprising administering a composition comprising one or more gap junction modulating agents and a pharmaceutically acceptable carrier or diluent. In one embodiment, the arterial ulcer or ulcerations are caused by complete or partial arterial blockage.

The invention also includes methods of treating and/or preventing, in whole or in part, by venous ulcers or ulcerations comprising administering a composition comprising one or more gap junction modulating agents and a pharmaceutically acceptable carrier or diluent. In one embodiment, the venous ulcer or ulcerations are caused by malfunction of the venous valve and the associated vascular disease.

The invention also includes methods of treating and/or preventing, in whole or in part, by venous stasis ulcers or ulcerations comprising administering a composition comprising one or more gap junction modulating agents and a pharmaceutically acceptable carrier or diluent. In one embodiment, the venous stasis ulcer or ulcerations are caused by chronic passive venous congestion of the lower extremities and/or the resulting local hypoxia.

The invention also includes methods of treating and/or preventing, in whole or in part, by diabetic ulcers, including diabetic foot ulcers or ulcerations, comprising administering a composition comprising one or more gap junction modulating agents and a pharmaceutically acceptable carrier or diluent. In one embodiment, the diabetic foot ulcer or ulcerations are caused by neurological and/or vascular complications of diabetes.

The invention also includes methods of treating and/or preventing, in whole or in part, traumatic ulcers or ulcerations comprising administering a composition comprising one or more gap junction modulating agents and a pharmaceutically acceptable carrier or diluent. In one embodiment, the traumatic ulcer or ulcerations are associated with traumatic injuries to the body.

The invention also includes methods of treating and/or preventing, in whole or in part, burn ulcers or ulcerations comprising administering a composition comprising one or more gap junction modulating agents and a pharmaceutically acceptable carrier or diluent. In one embodiment, the burn ulcer or ulcerations are associated with burn injuries to the body.

The invention also includes methods of treating and/or preventing, in whole or in part, decubitus ulcers or ulcerations comprising administering a composition comprising one or more gap junction modulating agents and a pharmaceutically acceptable carrier or diluent.

The invention also includes methods of treating and/or preventing, in whole or in part, infectious ulcers or ulcerations comprising administering a composition comprising one or more gap junction modulating agents and a pharmaceutically acceptable carrier or diluent.

The invention also includes methods of treating and/or preventing, in whole or in part, pyoderma gangrenosum comprising administering a composition comprising one or more gap junction modulating agents and a pharmaceutically acceptable carrier or diluent.

The invention also includes methods of treating and/or preventing, in whole or in part, dehiscent wounds comprising administering a composition comprising one or more gap junction modulating agents and a pharmaceutically acceptable carrier or diluent.

The invention also includes methods of treating and/or preventing, in whole or in part, persistent epithial defects comprising administering a composition comprising one or more gap junction modulating agents and a pharmaceutically acceptable carrier or diluent.

The invention also includes methods of treating and/or preventing, in whole or in part, pressure ulcer or ulcerations comprising administering a composition comprising one or more gap junction modulating agents and a pharmaceutically acceptable carrier or diluent. In certain embodiments said pressure ulcer or ulceration is a AHCPR stage 1, stage 2, stage 3, and /or stage 4 ulcer or ulcerations.

In yet a further aspect, the invention provides a method of promoting wound healing in a patient which comprises the step of administering a composition comprising one or more gap junction modulating agents to a wound that does not heal at an expected rate in an amount effective to close a gap junction or hemichannel at and/or immediately adjacent the site of said wound.

In a further aspect, the invention provides a method of reducing inflammation in a patient which comprises the step of administering a composition comprising one or more gap junction modulating agents to a wound that does not heal at an expected rate in an amount effective to close a gap junction or hemichannel at and/or immediately adjacent the site of said wound.

In yet a further aspect, the invention provides a method of promoting wound healing in a patient which comprises the step of administering a composition comprising one or more gap junction modulating agents to a wound that does not heal at an expected rate in an amount effective to block a gap junction or hemichannel at and/or immediately adjacent the site of said wound.

In a further aspect, the invention provides a method of reducing inflammation in a patient which comprises the step of administering a composition comprising one or more gap junction modulating agents to wound that does not heal at an expected rate in an amount effective to block a gap junction or hemichannel at and/or immediately adjacent the site of said wound.

In yet a further aspect, the invention provides a method of promoting wound healing in a patient which comprises the step of administering a composition comprising one or more gap junction modulating agents to wound that does not heal at an expected rate in an amount effective to prevent or decrease, in whole or in part, gap junction or hemichannel activity at and/or immediately adjacent the site of said wound.

In a further aspect, the invention provides a method of reducing inflammation in a patient which comprises the step of administering a composition comprising one or more gap junction modulating agents to wound that does not heal at an expected rate in an amount effective to prevent or decrease, in whole or in part, gap junction or hemichannel activity at and/or immediately adjacent the site of said wound.

In yet a further aspect, the invention provides a method of promoting wound healing in a patient which comprises the step of administering a composition comprising one or more gap junction modulating agents to wound that does not heal at an expected rate in an amount effective to prevent or decrease, in whole or in part, the opening of a gap junction or hemichannel at and/or immediately adjacent the site of said wound.

In a further aspect, the invention provides a method of reducing inflammation in a patient which comprises the step of administering a composition comprising one or more gap junction modulating agents to wound that does not heal at an expected rate in an amount effective to decrease or prevent, in whole or in part, the opening of a gap junction or hemichannel at and/or immediately adjacent the site of said wound.

In yet a further aspect, the invention provides a method of promoting wound healing in a patient which comprises the step of administering a composition comprising one or more gap junction modulating agents to wound that does not heal at an expected rate in an amount effective to prevent or decrease, in whole or in part, extracellular hemichannel communication at and/or immediately adjacent the site of said wound.

In a further aspect, the invention provides a method of reducing inflammation in a patient which comprises the step of administering a composition comprising one or more gap junction modulating agents to wound that does not heal at an expected rate in an amount effective to decrease or prevent, in whole or in part, extracellular hemichannel communication at and/or immediately adjacent the site of said wound.

According to one aspect, the gap junction modulating agent promotes wound healing by regulating epithelial basal cell division and growth by promoting re-epithelialization.

In one aspect the invention is directed to a method of promoting or improving wound healing in a subject, comprising administration of one or more gap junction modulating agent, in an amount effective to regulate epithelial basal cell division and growth. In one embodiment, the gap junction modulating agent is a anti-connexin peptide or peptidomimetic effective to regulate epithelial basal cell division and growth. In one embodiment, the gap junction modulating agent is a connexin 26 gap junction modulating agent, a connexin 43 gap junction modulating agent, or a mixture thereof.

In one aspect the invention is directed to a method of promoting or improving wound healing, comprising administration of one or more gap junction modulating agent, in an amount effective to regulate outer layer keratin secretion. In one embodiment, the gap junction modulating agent is a anti-connexin peptide or peptidomimetic effective to regulate outer layer keratin secretion. In one embodiment, the gap junction modulating agent is a connexin 43 gap junction modulating agent, a 31.1 gap junction modulating agent, or a mixture thereof.

### Kits and Articles of Manufacture

In one aspect, the invention provides a kit for treating wounds that do not heal at the expected rate.

The kit may include one or more compositions described herein. For example, the kit may include a composition comprising an effective amount of one or more gap junction modulating agents. In one embodiment, the kit comprises a composition that comprises an effective amount of one or more polypeptides. In one embodiment, the kit comprises a composition that comprises an effective amount of one or more polypeptide homologues. In one embodiment, the kit comprises a composition that comprises an effective amount of one or more connexin phosphorylation compounds.

In another aspect, the invention includes an article of manufacture comprising a vessel containing an effective amount of one or more gap junction modifying agents and instructions for use, including use for the treatment of a subject having a wound that does not heal at the expected rate.

Thus, in accordance with the invention, there are provided methods, compositions, forms, formulations, kits, and articles of manufacture by which cell-cell communication and/or hemichannel communication, docking or opening can be downregulated in a transient and site-specific manner. The formulations therefore have application in methods of therapy and in other treatments.

Various aspects of the invention will now be described with reference to the following examples which will be understood to be provided by way of illustration only and not to constitute a limitation on the scope of the invention.

### EXAMPLES

### EXAMPLE 1

### AFFINITY BINDING ASSAYS FOR PEPTIDES

Pull-down assays may be used to verify protein-peptide interaction. In this assay the test gap junction modulating agent (e.g. peptide) is tagged with a protein-reactive or fusion tag i.e. GST (Glutathione-S-Transferase), which is used to capture and 'pull-down' a protein-binding partner via attachment to a cellulose, agarose or nickel bead. Following elution of the complex utilizing either SDS-PAGE loading buffer or alternatively competitive analyte elution, the complex is visualised by running on an SDS-PAGE gel and using Western Analysis detection methods. Art known methods of performing binding assays are described in Einarson, M.B. and Orlinick, J.R., "Identification of Protein-Protein Interactions with Glutathione S-Transferase Fusion Proteins" in Protein-Protein Interactions: A Molecular Cloning Manual, Cold Spring Harbor Laboratory Press, pp. 37-57 (2002); Einarson, M.B., "Detection of Protein-Protein Interactions Using the GST Fusion Protein Pulldown Technique," in Molecular Cloning: A Laboratory Manual, 3rd Edition, Cold Spring Harbor Laboratory Press, pp.18.55-18.59 (2001); and Vikis, H.G. and Guan, K.L., "Glutathione-S-Transferase-Fusion Based Assays for Studying Protein-Protein Interactions," in Protein-Protein Interactions, Methods and Applications, Methods in Molecular Biology, 261, Fu, H. Ed. Humana Press, Totowa, N.J., pp. 175-186 (2004), each of which is hereby incorporated by reference in its entirety.

Interactions and affinity of proteins and test peptides are assessed using surface plasmon resonance technology which enables these interactions to be measured in real time (available through BIAcore). This approach has the advantage of detecting low affinity protein interactions. Surface plasmon resonance relies on an optical phenomenon that is used to measure changes in the solution concentration of molecules at a biospecific surface. This signal arises in thin metal films under conditions of total internal reflection. This signal depends on the refractive index of solutions in contact with the surface. Molecules in solution exhibit changes in refractive index and thus give rise to a measurable signal if a biospecific interaction occurs.

Typically, the protein is immobilized by one of several possible methods onto a carboxymethylated dextran-gold surface. The interacting peptide of interest is injected over the surface and the kinetics of binding are measured in real time. Art known methods of performing binding assays are described in Schuck, P., "Reliable determination of binding affinity and kinetics using surface plasmon resonance biosensors", Currrent Opinion in Biotechnology, 8(4):498-502 (1997); and Zhang, X., Oglesbee, M. "Use of surface plasmon resonance for the measurement of low affinity binding interactions between HSP72 and measles virus nucleocapsid protein." Biological Procedures Online. 5(1): 170-181 (2003).

### EXAMPLE 2

### FUNCTIONAL ASSAYS

Functional assays can be used to determine whether test gap junction modulating peptidomimetics are able to block the opening of gap junctions or hemichannels. HeLa human cervical cancer cell line is stably transfected with Cx43, Cx45, or another particular connexin of interest. The cells are incubated in a zero calcium solution (HBSS-HEPES containing 1mM EGTA), which has been shown to activate connexin hemichannels (See Braet, K., et al., "Pharmacological sensitivity of ATP release triggered by photoliberation of inositol-1,4,5-trisphosphate and zero extracellular calcium in brain endothelial cells," Journal of Cellular Physiology, 197(2): p. 205-213 (2003); DeVries, S.H. and E.A. Schwartz, "Hemi-gap-junction channels in solitary horizontal cells of the catfish retina," Journal of Physiology 445: p. 201-230 (1992); and Li, H., et al., "Properties and regulation of gap junctional hemichannels in the plasma membranes of cultured cells," Journal of Cell Biology 134(4): p. 1019-1030 (1996)). Cells are be incubated for 30 minutes in a solution of HBSS-HEPES containing 1mM EGTA and 2 mM propidium iodide. Propidium iodide is a fluorescent dye which is membrane impermeable but because of its small molecular weight is able to enter through gap junction/hemichannels. Propidium iodide uptake will be determined using fluorescence microscopy. Cells are incubated with propidium iodide in the presence of the peptidomimetics to determine if these can prevent dye uptake.

### EXAMPLE 3

### PEPTIDOMIMETIC DESIGN AND TESTING

Peptidomimetics can be designed to have the same amino acid sequence as connexin 43 extracellular loop regions believed to be involved in the connexon docking process (Foote et al., J Cell Biol 140(5):1187-97, (1998)). Some peptides include amino acids matching the outer portions of the alpha helical transmembrane subunits, which may show enhanced functional inhibition. Not all of these peptides are necessarily connexin specific due to the conservation of connexin sequences in the extracellular loop regions.

Peptides or variants thereof, can be synthesized *in vitro, e.g.,* by the solid phase peptide synthetic method as described in Stewart et al., Solid Phase Peptide Synthesis, W. H. Freeman Co., San Francisco (1969); Merrifield, J. Am. Chem. Soc. 85 2149 (1963); Meienhofer in "Hormonal Proteins and Peptides," ed.; C.H. Li, Vol.2 (Academic Press, 1973), pp.48-267; and Bavaay and Merrifield, "The Peptides," eds. E. Gross and F. Meienhofer, Vol.2 (Academic Press, 1980) pp.3-285. These peptides can be further purified by fractionation on immunoaffinity or ion-exchange columns; ethanol precipitation; reverse phase HPLC; chromatography on silica or on an anion-exchange resin such as DEAE; chromatofocusing; SDS-PAGE; ammonium sulfate precipitation; gel filtration using, for example, Sephadex G-75; ligand affinity chromatography; or crystallization or precipitation from non-polar solvent or nonpolar/polar solvent mixtures.

Two functional tests can be carried out using test peptides. These functional test were (i) blockage of dye (Lucifer Yellow) uptake by cells in spinal cord slices, and (ii) prevention of oedema in spinal cord segments (using connexin 43 specific antisense odn as a positive control). Peptides are synthesised by Sigma-Genosys (Australia).

### Blockage of dye (Lucifer Yellow) uptake by cells in spinal cord slices.

Lucifer Yellow is a small water soluble, fixable, dye able to pass from cell to cell via gap junction channels, but not across the cell membrane. The addition of Lucifer Yellow to the extracellular medium makes it possible to check for the presence of open gap junction/ hemichannels. The dye will appear in the cytoplasm of cells which are expressing open channels.

Wistar p7 rats are anesthetized with carbon dioxide and immediately decapitated. The spinal cord is excised and transferred to cold Hank's balanced salt solution (HBSS) at pH 7.4. Excess branch nerves and ligaments are removed and the cord transferred to a manual tissue chopper and a series of 500 micron thick slices cut. The damage caused by the slicing induces connexin 43 upregulation through the entire slice (exacerbated by the gap junction mediated bystander effect), and leads to the expression of connexin hemichannels. Slices are placed onto 3 cm diameter Millipore inserts in 24 well plates and cultured in the presence of a peptidomimetics in the media. The final concentration for all 9 peptides tested was 500 micromolar. Controls were; no peptide added, or with 1% ethanol or 1% DMSO added as some peptides may be re-dissolved in these compounds (peptides were received lyophilised).

Slices are incubated for four hours at 37 degrees C, and then 2.5mg per ml Lucifer Yellow is added to each well for 30 minutes (in the dark). The tissues are then rinsed twice in PBS, with three further 10 minute washes, and the slices fixed with 4% paraformaldehyde. They were then viewed using a Leica TCS4D laser scanning confocal microscope to assess dye uptake into cells, or not. Efficacy is measured by the amount of dye uptake into each sample.

### Prevention of oedema in spinal cord

The system described in Example 1 of WO2000/44409 to Becker, D. and Green. C., entitled "Formulations Comprising Antisense Nucleotides to Connexins" is used to examine the effects of test peptides on cultured spinal cord segments to test their ability to block swelling.

DMSO is used as a control. 5mm long spinal cord segments were placed in separate wells of a 24 well plate in HBSS. The segments are held to the bottom of the well using a small drop of Superglue. The HBSS is removed and 500 micromolar test peptide (final concentration) added to media (no peptide for media alone or DMSO controls). The plates are incubated for 24 hours, the media removed and the tissue fixed with Bouin's fixative for 24 hours. Analysis involves photographing cord segments from above, with Image J to calculate the total area of the cord segment compared with the area of swelling at the cut ends of the segments. Swelling (oedema) is calculated as (cultured area - original area divided by original area) to give % swelling. Single factor Analysis of Variance is used to determine statistical significance, with cut-off level for significance at p=0.05.

Determining the most effective concentration of the test peptide for blocking of oedema in spinal cord segments is carried out using the same protocol using a dose response curve. One exemplary dose response curve is determined with final concentration of peptides used at 5, 10, 50 250 and 500 micromolar.

### EXAMPLE 4

The method and compositions disclosed herein are used to treat a human subject with a chronic wound (e.g., a vasculitic ulcer).

A human subject with diabetes, or underlying peripheral vascular or arterial disease first presented for complications arising from a non-closing leg wound. The wound is treated with a suitable dose or doses of an anti-connexin peptide SRPTEKTIFII (SEQ ID NO: 7), e.g., 100-500 micrograms. 2 mL of 20 µM preparation of SEQ ID NO: 7 in pluronic gel (e.g., total dose ∼200-400 µg) based on a wound size of approximately 7 cm x 5 cm (about 35 cm2) with a depth of approximately 3-4 mm is administered (other appropriate dosages to be administered can be readily determined by a skilled practitioner in accordance with the wound size).

The wound site is dressed and covered for a period of 7 days. The wound is uncovered on Day 7 and the wound healing results are assessed.

The treatment outlined above is repeated (in appropriate dosage) as necessary or desired. Following this treatment, the wound and the wound appearance is assessed. The patient is evaluated again at two weeks, one month, and/or two months and the wound (reduction if any) is again evaluated.

### EXAMPLE 5

The method and compositions disclosed herein are used to treat a human subject with a chronic venous leg ulceration.

Human test subjects are grouped according to ulcer size and minimum and maximum ulcer areas (e.g., 2 cm² and 50 cm²). Patient's resting ankle brachial doppler arterial pressure index are determined as a baseline (e.g. equal to or greater than 0.80).

All patients receive compression bandaging. The area of the ulcer is determined by tracing, and suitable dosages of test anti-connexin peptide SRPTEKTIFII (SEQ ID NO: 7) in a pluronic gel preparation, e.g., 100-500 micrograms, is administered

The patient is asked to lie recumbent on the examination couch while the test preparation is applied to the surface of the wound, and to remain there for 15 minutes before compression bandaging is applied.

The wound is uncovered on Day 7 and the wound healing results are assessed. Wound fluids and blood samples are analyzed for relevant wound healing biomarkers using bioassays.

The treatment outlined above is repeated (in appropriate dosage). Following this treatment, the wound and the wound appearance is assessed. This course is repeated weekly or bi-weekly, or as appropriate given the state of healing, until wound closure.

### EXAMPLE 6

The following are used to determine therapeutic efficacy of sequential administration of exemplary preparations in accelerating the healing rate of diabetic and other chronic ulcers.

The primary efficacy endpoint is the percentage of patients achieving full wound closure within 12-20 weeks. Secondary endpoints include the time to 100% closure, time to 80% closure, time to 50% closure, and the amount of wound closure as a percentage change from the baseline wound size at 3, 5, 10, 15, and 20 weeks. Kaplan-Meier survival analysis techniques are utilized to examine the time-to-event endpoints.

All patients receive a regimen of standard diabetic (or other) ulcer care consisting of initial sharp debridement, wound cleansing, wound dressing, and wound pressure offloading. The ulcer is treated by an initial sharp debridement and wound cleansing. 100-500 micrograms of an anti-connexin peptide SRPTEKTIFII (SEQ ID NO: 7) in a pluronic gel preparation is administered sequentially. The wound is dressed with a non-stick bandage and pressure bandage.

Wounds are evaluated twice a week for up to 12-20 weeks or until wound closure, whichever is earlier. Patients are removed from the study if they developed a clinical infection or if the wound condition significantly deteriorated. At each wound evaluation (twice weekly), the wound perimeter is traced for determination of wound area, and the wound is photographed with a digital camera. Blood chemistry and hematology tests are performed at patient enrollment, and at weeks 5, 10, 15, and 20. A radiographic assessment may be conducted every 5 weeks to study effects on underlying bone composition.

### EXAMPLE 7

Anti-connexin agent is conveniently formulated in a form suitable for administration according to the methods of the present invention.

Suitable formulations include a mixture of the following formulating agents. The amount of the individual anti-connexin agent or agents and formulating agents will depend of the particular use intended.

| |
|---|
| ASO in PBS |
| Polyquaternium 10 |
| HEC/HPMC/CMC |
| Na Hyaluronate |
| Tween 20 |
| Poloxamer 188 |
| Pluronic 87 NF |
| SLES |
| Poly L-lysine/Polyethylene Imine |
| Benzalkonium chloride |
| Methyl paraben |
| Propyl paraben |
| Propylene Glycol |
| 10mM Phosphate Buffer |

All patents, publications, scientific articles, web sites, and other documents and materials referenced or mentioned herein are indicative of the levels of skill of those skilled in the art to which the invention pertains, and each such referenced document and material is hereby incorporated by reference to the same extent as if it had been incorporated by reference in its entirety individually or set forth herein in its entirety. Applicants reserve the right to physically incorporate into this specification any and all materials and information from any such patents, publications, scientific articles, web sites, electronically available information, and other referenced materials or documents.

The specific methods and compositions described herein are representative of preferred embodiments and are exemplary and not intended as limitations on the scope of the invention. Other objects, aspects, and embodiments will occur to those skilled in the art upon consideration of this specification, and are encompassed within the spirit of the invention as defined by the scope of the claims. It will be readily apparent to one skilled in the art that varying substitutions and modifications may be made to the invention disclosed herein without departing from the scope and spirit of the invention. The invention illustratively described herein suitably may be practiced in the absence of any element or elements, or limitation or limitations, which is not specifically disclosed herein as essential. Thus, for example, in each instance herein, in embodiments or examples of the present invention, any of the terms "comprising", "consisting essentially of', and "consisting of" may be replaced with either of the other two terms in the specification. Also, the terms "comprising", "including", containing", etc. are to be read expansively and without limitation. The methods and processes illustratively described herein suitably may be practiced in differing orders of steps, and that they are not necessarily restricted to the orders of steps indicated herein or in the claims. It is also that as used herein and in the appended claims, the singular forms "a," "an," and "the" include plural reference unless the context clearly dictates otherwise. Under no circumstances may the patent be interpreted to be limited to the specific examples or embodiments or methods specifically disclosed herein. Under no circumstances may the patent be interpreted to be limited by any statement made by any Examiner or any other official or employee of the Patent and Trademark Office unless such statement is specifically and without qualification or reservation expressly adopted in a responsive writing by Applicants.

The terms and expressions that have been employed are used as terms of description and not of limitation, and there is no intent in the use of such terms and expressions to exclude any equivalent of the features shown and described or portions thereof, but it is recognized that various modifications are possible within the scope of the invention as claimed. Thus, it will be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

Other embodiments are within the following claims. In addition, where features or aspects of the invention are described in terms of Markush groups, those skilled in the art will recognize that the invention is also thereby described in terms of any individual member or subgroup of members of the Markush group.

### ASPECTS OF THE INVENTION

1. A method of treating a subject having a chronic wound comprising administration of a composition comprising an effective amount of a connexin 43 gap junction modulating agent to the wound.
2. A method of 1, wherein the chronic wound is a pressure ulcer.
3. A method of 1, wherein the chronic wound is a decubitus ulcer.
4. A method of 1, wherein the chronic wound is an arterial ulcer.
5. A method of 1, wherein the chronic wound is a venous ulcer.
6. A method of 1, wherein the chronic wound is a venous stasis ulcer.
7. A method of 1, wherein the chronic wound is a diabetic foot ulcers
8. A method of 1, wherein the chronic wound is a traumatic ulcers
9. A method of 1, wherein the chronic wound is a burn ulcer.
10. A method of treating a subject having a wound that does not heal at the expected rate, which comprises administration of an effective amount of a connexin 43 gap junction modulating agent to the wound.
11. A method of 10 wherein the wound is a delayed healing wound or an incompletely healing wound.
12. A method of treating a subject having a dehisecent wound comprising administration of a composition comprising an effective amount of a connexin 43 gap junction modulating agent to the wound.
13. A method of any of 1, 10 or 12, wherein the gap junction modulating agent prevents or decreases, in whole or in part, flow of molecules through a gap junction.
14. A method of any of 1, 10 or 12, wherein the gap junction modulating agent prevents or decreases, in whole or in part, flow of molecules through a hemichannel.
15. A method of any of 1, 10 or 12, wherein the gap junction modulating agent closes, in whole or in part, a gap junction.
16. A method of any of 1, 10 or 12, wherein the gap junction modulating agent closes, in whole or in part, a hemichannel.
17. A method of any of 1, 10 or 12, wherein the gap junction modulating agent blocks, in whole or in part, a gap junction.
18. A method of any of 1, 10 or 12, wherein the gap junction modulating agent blocks, in whole or in part, a hemichannel.
19. A method of any of 1, 10 or 12, wherein the gap junction modulating agent induces closure, in whole or in part, of a gap junction.
20. A method of any of 1, 10 or 12, wherein the gap junction modulating agent induces closure, in whole or in part, of a hemichannel.
21. A method of any of 1, 10 or 12, wherein the gap junction modulating agent decreases or prevents, in whole or in part, the opening of gap junction.
22. A method of any of 1, 10 or 12, wherein the gap junction modulating agent decreases or prevents, in whole or in part, the opening of a hemichannel.
23. A method of any of 1, 10 or 12, wherein the gap junction modulating agent prevents or decreases the activity or function of a gap junction.
24. A method of any of 1, 10 or12, wherein the gap junction modulating agent prevents or decreases the activity or function of a hemichannel.
25. A method of any of 1, 10 or 12, wherein the gap junction modulating agent is a peptide compound.
26. A method of 25 wherein the peptide compound is a peptidomimetic.
27. A method of 25, wherein the gap junction modulating agent is an antibody or antigen binding fragment thereof.
28. A method of 27 wherein the antibody is a monoclonal antibody.
29. A method of 27, wherein the antigen binding fragment is an F(v), Fab, Fab' or F(ab')₂ anti-connexin 43 antibody fragment.
30. A method of 27, wherein the antibody is a chimeric or humanized antibody.
31. A method of 27, wherein the antibody fragment is a chimeric or humanized antibody fragment.
32. A method of any of 1, 10 or 12, wherein the gap junction modulating agent is a gap junction phosphorylation inducing agent.
33. A method of any of 1, 10 or 12, wherein the gap junction modulating agent is formulated for topical administration.
34. A method of any of 1, 10 or 12, wherein the gap junction modulating agent is formulated as a gel.
35. A method of any of 1, 10 or 12, wherein the subject is a human.
36. A method of any of 1, 10 or 12, wherein the subject is a non-human animal.
37. A method of 36, wherein the non-human animal is a sports animal.
38. A method of 37, wherein the sports animal is a horse.
39. A method of 37, wherein the sports animal is a dog.
40. A method of 36, wherein the non-human animal is a pet animal.
41. A method of 40, wherein the pet animal is a dog.
42. A method of 40, wherein the pet animal is a cat.
43. A method of 1, wherein the chronic wound is a persistent epithelial defect.

## Claims

1. A connexin 43 gap junction modulating agent composition comprising a hemichannel blocking polypeptide for use in a method of treating a subject having:
(a) a chronic wound;
(b) a wound that does not heal at the expected rate; or
(c) a dehiscent wound,
wherein the method comprises administering:
(i) the connexin 43 gap junction modulating agent composition at 20 µM, 30 µM, 40 µM, 50 µM, 60 µM, 70 µM, 80 µM, 90 µM, 100 µM, 10-200 µM, 200-300 µM, 300-400 µM, 400-500 µM, 500-600 µM, 600-700 µM, 700-800 µM, 800-900 µM, or 900-1000 µM or 1000-1500 µM, or 1500-2000 µM or 2000-3000 µM, or up to 300 µM or up to 1000 µM, or up to 2000 µM or up to 3200 µM or more final concentration.

2. The composition of claim 1, for use according to claim 1, wherein
(i) the chronic wound is a pressure ulcer, a decubitus ulcer, an arterial ulcer, a venous ulcer, a venous stasis ulcer, a diabetic foot ulcer, a traumatic ulcer or a burn ulcer; or
(ii) the wound that does not heal at the expected rate is a delayed healing wound or an incompletely healing wound.

3. The composition of any one of the preceding claims, for use according to any one of the preceding claims, wherein the connexin 43 gap junction modulating agent is a peptidomimetic or an antibody or antigen binding fragment thereof, wherein the connexin 43 peptidomimetic optionally comprises SEQ ID NO: 6, SEQ ID NO: 7, SEQ ID NO: 50 or SEQ ID NO:55.

4. The composition of any one of the preceding claims, for use according to any one of the preceding claims, wherein the method further comprises administering:
(ii) a connexin 31.1 gap junction modulating agent composition comprising a hemichannel blocking polypeptide at 20 µM, 30 µM, 40 µM, 50 µM, 60 µM, 70 µM, 80 µM, 90 µM, 100 µM, 10-200 µM, 200-300 µM, 300-400 µM, 400-500 µM, 500-600 µM, 600-700 µM, 700-800 µM, 800-900 µM, or 900-1000 µM or 1000-1500 µM, or 1500-2000 µM or 2000-3000 µM, or up to 300 µM or up to 1000 µM, or up to 2000 µM or up to 3200 µM or more final concentration.

5. The composition of claim 4, for use according to claim 4, wherein the connexin 31.1 gap junction modulating agent is a peptidomimetic or an antibody or antigen binding fragment thereof, wherein the peptidomimetic optionally comprises SEQ ID NO: 31, SEQ ID NO: 45, or SEQ ID NO: 60.

6. The composition of claim 3 or 5, for use according to claim 3 or 5, wherein:
(a) the antibody is a monoclonal antibody;
(b) the antigen binding fragment is an F(v), Fab, Fab' or F(ab')2 antibody fragment;
(c) the antibody is a chimeric or humanized antibody; or
(d) the antibody fragment is a chimeric or humanized antibody fragment.

7. The composition of any one of the preceding claims, for use according to any one of the preceding claims, wherein the gap junction modulating agent(s):
(a) prevent or decrease, in whole or in part, flow of molecules through a gap junction or a hemichannel;
(b) close, in whole or in part, a gap junction or a hemichannel;
(c) block, in whole or in part, a gap junction or a hemichannel; or
(d) decrease or prevent, in whole or in part, the opening of gap junction or a hemichannel.

8. The composition of any one of the preceding claims, for use as described in any one of the preceding claims, which is formulated for topical administration.

9. The composition of any one of the preceding claims, for use according to any one of the preceding claims, which is formulated as a gel.

10. The composition of any one of the preceding claims, for use according to any one of the preceding claims, wherein the subject is a human.

11. The composition of any one of the preceding claims, for use according to any one of the preceding claims, wherein the wound is a skin wound.

12. The composition of any one of the preceding claims, for use according to any one of the preceding claims, where the agents are administered with a biological dressing or biosynthetic dressing.

13. The composition according to claim 1, for use according to claim 1, wherein the connexin 43 gap junction modulating agent is administered more than once.

14. The composition according to claim 4, for use according to claim 4, wherein the connexin 43 gap junction modulating agent and connexin 31.1 gap junction modulating agent are administered within at least about:
(i) one-half hour of each other;
(ii) one hour of each other;
(iii) one day of each other; or
(iv) one week of each other.

15. The composition according to claim 1 or 4, wherein connexin 43 gap junction modulating agent and connexin 31.1 gap junction modulating agent are applied:
(a) more than once;
(b) about once per week;
(c) when wound healing is stalled or slowing;
(d) every 1 to 7 days;
(e) bi-weekly; or
(f) monthly.
